(19) Europäisches Patentamt
European Patent Office
Office européen des brevets

(11) EP 2 651 410 B1

(12) # EUROPÄISCHE PATENTSCHRIFT

(45) Veröffentlichungstag und Bekanntmachung des Hinweises auf die Patenterteilung:
**14.10.2020 Patentblatt 2020/42**

(21) Anmeldenummer: **11799413.7**

(22) Anmeldetag: **14.12.2011**

(51) Int Cl.:
***A61K 9/70*** $^{(2006.01)}$ ***A61K 31/27*** $^{(2006.01)}$
***A61P 17/00*** $^{(2006.01)}$ ***A61P 25/16*** $^{(2006.01)}$
***A61P 25/28*** $^{(2006.01)}$

(86) Internationale Anmeldenummer:
**PCT/EP2011/072812**

(87) Internationale Veröffentlichungsnummer:
**WO 2012/080365 (21.06.2012 Gazette 2012/25)**

(54) **TRANSDERMALES THERAPEUTISCHES SYSTEM ZUR VERABREICHUNG EINES WIRKSTOFFS**

TRANSDERMAL THERAPEUTIC SYSTEM FOR ADMINISTERING AN ACTIVE SUBSTANCE

SYSTÈME THÉRAPEUTIQUE TRANSDERMIQUE POUR L'ADMINISTRATION D'UN PRINCIPE ACTIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **14.12.2010 EP 10194968**

(43) Veröffentlichungstag der Anmeldung:
**23.10.2013 Patentblatt 2013/43**

(73) Patentinhaber: **Luye Pharma AG**
**83714 Miesbach (DE)**

(72) Erfinder:
- **LANGER, Britta**
  **10965 Berlin (DE)**
- **SCHURAD, Bjoern**
  **81667 München (DE)**
- **PRINZ, Heike**
  **82008 Unterhaching (DE)**

(74) Vertreter: **Kalhammer, Georg et al**
**Lederer & Keller**
**Patentanwälte Partnerschaft mbB**
**Unsöldstrasse 2**
**80538 München (DE)**

(56) Entgegenhaltungen:

| | |
|---|---|
| **EP-A1- 2 016 939** | **WO-A1-2007/064407** |
| **WO-A1-2009/009651** | **WO-A2-2009/026135** |
| **DE-A1- 19 918 106** | **US-A1- 2008 175 890** |

Bemerkungen:
Die Akte enthält technische Angaben, die nach dem Eingang der Anmeldung eingereicht wurden und die nicht in dieser Patentschrift enthalten sind.

EP 2 651 410 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gegenstand der vorliegenden Anmeldung ist ein System zur transdermalen Verabreichung von Rivastigmin, dessen physiologisch verträglichem Salz, Hydrat, Solvat oder Derivat, das für einen therapeutischen Anwendungszeitraum von mehreren Tagen geeignet ist.

Bei Rivastigmin handelt es sich um das Phenylcarbamat (S)-N-ethyl-3-[(1-dimethylamino)ethyl]-N-methyl-phenyl-carbamat der Formel I.

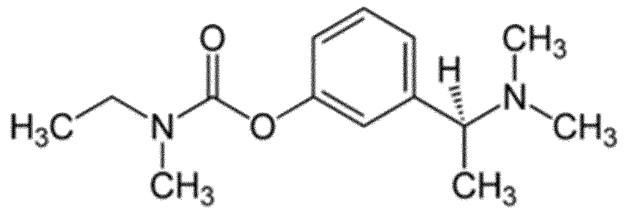

(I)

Es ist ein im zentralen Nervensystem wirkender Cholinesteraseinhibitor und ist daher ein Wirkstoff zur Behandlung von Alzheimer und Parkinsondemenz.

Rivastigmin kann als freie Base, aber auch als Säureadditionssalz, Hydrat, Solvat oder als sonstiges Derivat vorliegen. Diese Derivate sind, sofern nicht anders beschrieben, in der vorliegenden Erfindung mit der Bezeichnung "Rivastigmin" umfasst.

Eine bevorzugte Darreichungsform von Rivastigmin ist die perkutane Verabreichung mittels eines transdermalen therapeutischen Systems, also ein Transdermalpflaster. Ein Transdermalpflaster ist typischerweise eine kleine selbstklebende Bandage, welche den abzugebenden Wirkstoff enthält. Diese Bandagen können verschiedene Formen und Größen haben. Der einfachste Typ ist ein Klebe-Monolith, welcher einen Wirkstoffvorrat auf einem Träger (Deckschicht) umfasst. Dieser Wirkstoffvorrat wird typischerweise in Form einer Wirkstoffschicht aus dem Wirkstoff in einem pharmazeutisch akzeptablen, druckempfindlichen Klebstoff oder Haftkleber gebildet und steht mit der Hautoberfläche in Kontakt, wodurch der Wirkstoff durch transdermale Diffusion in den Körper des Patienten abgegeben wird.

Komplexere Pflaster sind Mehrfachlaminate oder Pflaster mit Wirkstoffvorrat, in welchen zwischen der Wirkstoffschicht und der Haut eine weitere Klebeschicht angeordnet sein kann. Eine Darreichungsform über Transdermalpflaster von Rivastigmin wurde bereits im Grundpatent zu Rivastigmin, der GB 2203040 beschrieben. Das darin offenbarte Transdermalpflaster besteht aus einer Deckschicht und einer die Wirkstoffschicht bildenden Schicht. Neben dem Wirkstoff Rivastigmin sind in der Wirkstoffschicht ein hydrophiles Polymer, ein nicht-quellendes Acrylatpolymer und ein Weichmacher enthalten.

Nach Veröffentlichung der GB 2203040 wurden weitere transdermale therapeutische Systeme (TTS), die unter anderem Rivastigmin als Wirkstoff enthalten, entwickelt und beschrieben. In der WO 02/03969 ist ein TTS beschrieben, bei dem die wirkstoffenthaltende Matrixschicht zusätzlich hochdisperses Siliziumdioxid zur Erhöhung der Hautpermeation enthält.

In den Patentanmeldungen WO 2008/021113 und EP 2 016 939 werden Pflaster mit einem komplexeren Aufbau offenbart, welche eine Verabreichung verschiedener Wirkstoffe über einen Zeitraum von bis zu sieben Tagen gewährleisten sollen. Diese Pflaster enthalten zwischen der den Wirkstoff enthaltenden Schicht und einer Klebeschicht zusätzlich eine die Abgabe des Wirkstoffs steuernde Membran. In der Wirkstoffschicht des Pflasters sind aber freie Hydroxylgruppen vorhanden, da in der Wirkstoffschicht ein mehrwertiger Alkohol enthalten ist.

In der DE 199 18 106 enthält die Wirkstoffschicht ein haftklebendes Polymer, welches Acrylsäure oder Methacrylsäureeinheiten mit einem definierten Gehalt an Carboxylgruppen aufweist, wodurch die Wasseraufnahmefähigkeit sowie die Toleranz saurer Polyacrylhaftkleber gegenüber Feuchtigkeit erhöht werden soll.

Die WO 2007/064407 A1 offenbart ein TTS mit einer auf Silikon basierenden Klebeschicht, wodurch eine Verbesserung hinsichtlich der Klebeeigenschaften, der Verträglichkeit und der Sicherheit bei der Rivastigmintherapie erreicht werden soll. Gemäß WO 2007/064407 A1 ist es besonders bevorzugt, dass die Reservoirschicht ein Antioxidationsmittel enthält (Seite 7, 4. Absatz). Entsprechend enthalten alle Formulierungen in den Beispielen das Antioxidationsmittel Vitamin E. Das dort verwendete Durotak® 387-2353 ist ein Polyacrylat, das Carboxylgruppen aufweist. Die Reservoirschicht soll nach WO 2007/064407 A1 auch als Penetrationsverbesserer verschiedene Substanzen wie z. B. Glycerin, Fettsäuren, etc. enthalten (Seite 7, 5. Absatz). Diese Substanzen enthalten meist freie Hydroxyl- oder Carboxylgruppen, die damit in der Polymermatrix der Reservoirschicht vorhanden sind. WO 2007/064407 A1 beschäftigt sich nicht besonders mit der Stabilität von Rivastigmin. Insbesondere lehrt WO 2007/064407 A1 nicht, bestimmte Polymere für die Polymermatrix der Reservoirschicht auszuwählen, um einem Abbau von Rivastigmin vorzubeugen.

US 2008/0044461 A1 offenbart TTS-Formulierungen mit Donepezil (siehe Beispiele). Rivastigmin wird auch erwähnt (Anspruch 7). US 2008/0044461 A1 offenbart keine Wirkstoffschicht mit einer Polymermatrix. Vielmehr wird

die Freisetzung über eine Membran gesteuert (sog. Membranpflaster), nicht durch ein Polymergerüst, in das der Wirkstoff eingebettet ist (sog. Matrixpflaster). Zudem ist es ein essentielles Merkmal von US 2008/0044461 A1, dass die Reservoirschicht einen Gelbildner und einen Permeationsverstärker enthält (siehe Anspruch 1). Als Permeationsverstärker werden Alkohole eingesetzt (siehe [0053]). Als Gelbildner werden Cellulosepolymere eingesetzt (siehe [0055]). Sowohl bei den Permeationsverstärkern als auch bei den Gelbildnern handelt es sich somit um Verbindungen mit freien Hydroxylgruppen, die im TTS in der Reservoirschicht vorhanden sind.

US 2007/0259028 A1 offenbart TTS-Formulierungen mit Donepezil (siehe Beispiele). Rivastigmin wird auch erwähnt (Anspruch 3). Es ist ein essentielles Merkmal von US 2007/0259028 A1, dass die Reservoirschicht einen mehrwertigen Alkohol enthält, z. B. Glycerin. Gemäß US 2007/0259028 A1 sind also zwangsläufig freie Hydroxylgruppen in der Polymermatrix der Reservoirschicht vorhanden.

US 2004/0086552 A1 offenbart TTS-Formulierungen mit einem Wirkstoff, der aus einer sehr langen Liste ausgewählt sein kann (siehe [0070] bis [0095]). Es werden sowohl Matrixpflaster als auch "Membranpflaster" offenbart (siehe [0057] bzw. [0058]). Für die Matrixpflaster lehrt US 2004/0086552 A1 nicht, bestimmte Polymere für die Matrix auszuwählen, um den Wirkstoff zu stabilisieren.

US 6,689,379 B1 offenbart TTS-Formulierungen mit einer besonderen Klebeschicht. Rivastigmin wird auch als möglicher Wirkstoff erwähnt. Die Wirkstoffschicht soll vorzugsweise eine Verbindung mit Hydroxylgruppen enthalten, siehe Anspruch 10. US 6,689,379 B1 lehrt nicht, bestimmte Polymere für die Polymermatrix der Reservoirschicht auszuwählen, um einem Abbau von Rivastigmin vorzubeugen.

Das Patent EP 1 047 409 berichtet jedoch von einem generellen Problem bei der Verabreichung von Rivastigmin durch ein TTS. Es wurde gefunden, dass der Wirkstoff gerade in Gegenwart von Sauerstoff zersetzungsanfällig ist. In der transdermalen Zusammensetzung welche in der GB 2203040 offenbart ist, zersetzt sich gemäß der Offenbarung in EP 1 047 409 das Rivastigmin auch trotz der Bildung einer geschlossenen Polymermatrix um den Wirkstoff und einer luftdichten Verpackung der Zusammensetzung. In der EP 1 047 409 wird das Problem der geringen Stabilität von Rivastigmin dadurch gelöst, dass der pharmazeutischen Zusammensetzung ein Antioxidationsmittel zugesetzt wird.

Bisher erlauben kommerziell erwerbliche TDS mit Rivastigmin lediglich eine kontinuierliche Verabreichung über 24 Stunden. Eine Aufgabe der Erfindung ist die Steuerung der Freigabe über das verlängerte Zeitintervall. Bei der transdermalen Verabreichung von Rivastigmin über einen längeren Zeitraum muss sichergestellt sein, dass (1) eine ausreichende chemische Stabilität des Wirkstoffs Rivastigmin gewährleistet ist, (2) eine ausreichende physikalische Stabilität (v.a. hinsichtlich Cold flow) des Systems gewährleistet ist und (3) das System eine hinreichende Klebkraft über die Applikationszeit aufweist.

Eine weitere Aufgabe der vorliegenden Erfindung ist es, therapeutische Zusammensetzungen enthaltend Rivastigmin für die transdermale Verabreichung zu finden, die für einen therapeutischen Anwendungszeitraum von mehreren Tagen geeignet ist.

## Zusammenfassung der Erfindung

Es wurde gefunden, dass Rivastigmin in Transdermalpflastern hinreichend stabil ist, wenn die Polymermatrix der Wirkstoffschicht keine freien Hydroxylgruppen oder Carboxylgruppen enthält. Die vorliegende Erfindung beruht unter anderem darauf, spezielle Polymere für die Polymermatrix auszuwählen, um dadurch einen Abbau von Rivastigmin zu verhindern bzw. zu minimieren.

Die vorliegende Erfindung stellt daher ein hinreichend stabiles TTS enthaltend Rivastigmin und ein Verfahren zur Herstellung desselben bereit.

Ein erster Aspekt der vorliegenden Erfindung ist daher ein TTS zur Verabreichung von Rivastigmin für einen Anwendungszeitraum von mehreren Tagen, das folgende Komponenten umfasst:

a) eine Deckschicht,
b) eine auf der Deckschicht befindliche Wirkstoffschicht umfassend eine den Wirkstoff enthaltende Polymermatrix,
c) eine auf der Wirkstoffschicht befindliche, die Freisetzung des Rivastigmins kontrollierende Membran;
d) eine auf der Membran befindliche Klebeschicht umfassend einen Haftkleber und
e) eine auf der Klebeschicht befindliche Abziehschicht,

wobei das Polymer/die Polymere der Wirkstoffschicht keine freien Hydroxylgruppen oder Carboxylgruppen enthält/enthalten und das Polymer/die Polymere der Polymermatrix keine freien Hydroxylgruppen und keine freien Carboxylgruppen enthält/enthalten.

Weiterhin stellt die Erfindung die Verwendung von Polymeren oder Copolymeren, welche keine freien Hydroxylgruppen oder Carboxylgruppen aufweisen, in einem TTS enthaltend Rivastigmin und ein TTS zur Behandlung von Alzheimer und Parkinsondemenz bereit.

Während in der WO 2008/021113 die Klebstoffmaterialien Polyisobutylen, Polyacrylat und Klebstoffe auf Silikonbasis als gleichermaßen geeignet beschrieben werden, wurde vorliegend gefunden, dass sich Polyisobutylene als Haftkleber in der Klebeschicht besser eignen, und dass die Eigenschaften dieses Klebstoffs durch den Zusatz von Polybuten als Klebrigmacher weiter deutlich verbessert werden.

Ein zweiter Aspekt der vorliegenden Erfindung ist ein TTS zur Verabreichung von Rivastigmin durch die Haut umfassend eine Deckschicht, eine Wirkstoffschicht, welche das Rivastigmin enthält, eine verbesserte Klebeschicht und eine Abziehschicht, für die Verabreichung von Rivastigmin über einen Zeitraum von mindestens zwei (z. B. zwei oder drei) Tagen, wobei sich zwischen der Wirkstoffschicht und der Klebeschicht eine die Freisetzung von Rivastigmin steuernde Membran befindet. Ein zweiter Aspekt der vorliegenden Erfindung ist daher ein transdermales therapeutisches System zur Verabreichung eines Wirkstoffs durch die Haut, umfassend die zueinander in der folgenden Reihenfolge angeordneten Schichten:

a) eine Deckschicht,
b) eine Wirkstoffschicht umfassend eine den Wirkstoff enthaltende Polymermatrix,
c) eine die Freisetzung des Rivastigmins kontrollierende Membran;
d) eine Klebeschicht umfassend (1) einen Haftkleber, welcher aus einem Polyisobutylen oder einem Gemisch von mehreren Polyisobutylenen besteht und (2) einen Klebkraftverstärker, welcher aus Polybuten oder einem Gemisch von mehreren Polybutenen besteht, und
e) eine Abziehschicht, wobei der Wirkstoff Rivastigmin oder ein physiologisch verträgliches Salz, Hydrat, oder Solvat davon ist, dadurch gekennzeichnet, dass das Polymer/die Polymere der Polymermatrix keine freien Hydroxylgruppen und keine freien Carboxylgruppen enthält/enthalten, und dass die Wirkstoffschicht keine freien Hydroxylgruppen und keine freien Carboxylgruppen enthält.

Die oben genannten verschiedenen Aspekte der Erfindung können beliebig miteinander kombiniert werden. Der Wirkstoff im Sinne der vorliegenden Erfindung ist Rivastigmin oder ein physiologisch verträgliches Salz, Hydrat, Solvat oder Derivat davon.

**Ausführliche Beschreibung der Erfindung**

*Definitionen*

Ein "Antioxidationsmittel" im Sinne der vorliegenden Erfindung ist eine pharmazeutisch verträgliche Verbindung oder Zusammensetzung, die Oxidationsprozesse verlangsamt, hemmt, unterbricht und/oder aufhält. Antioxidationsmittel schließen insbesondere folgende Substanzen ein: Tocopherole und deren Ester, das Sesamol des Sesamöls, das Koniferylbenzoat des Benzoeharzes, die Nordihydroguajakharz- und -guajaretsäure (NDGA), die Gallate (Methyl-, Ethyl-, Propyl-, Amyl-, Butyl-, Lauryl- u.a. Gallate), das Butylhydroxdyanisol (BHT, auch Butyl-p-Kresol genannt); Ascorbinsäure und Salze und Ester davon, (z. B. Ascorbylpalmitat), Erythorbinsäure (iso-Ascorbinsäure) und Salze und Ester davon, Monothioglycerol, Natriumformaldehydsulfoxylat, Natriummetabisulfit, Natriumbisulfit, Natriumsulfit, Kaliummetabisulfit, butyliertes Hydroxyanisol, butyliertes Hydroxytoluol, Propionsäure.

Der Begriff "Tocopherol" schließt auch Tocopherol-Ester ein. Ein bekanntes Tocopherol ist $\alpha$-Tocopherol. Der Begriff "$\alpha$-Tocopherol" schließt Ester von $\alpha$-Tocopherol (z. B. $\alpha$-Tocopherol-Acetat) ein.

Eine "Polymermatrix" ist eine feste oder halbfeste Zusammensetzung mit dreidimensionaler Struktur, umfassend ein Polymer oder ein Polymergemisch. Die Polymermatrix wird auch als Polymergerüst bezeichnet, da die dreidimensionale Gerüst-Struktur in der Regel durch das Polymer oder Polymergemisch bereitgestellt wird. In die Polymermatrix können andere Stoffe, z. B. ein Wirkstoff, eingebettet sein. Vorzugsweise ist der Wirkstoff gleichmäßig in der Polymermatrix verteilt.

Im Folgenden werden die Merkmale der erfindungsgemäßen TTS detailliert beschrieben und, soweit es nicht ausdrücklich anders beschrieben ist, beziehen sich die jeweiligen Ausführungen zu den einzelnen Merkmalen auf sämtliche vorstehenden Aspekte der vorliegenden Erfindung.

Weiterhin können die vorstehenden Aspekte der vorliegenden Erfindung zu weiter bevorzugten Ausführungsformen beliebig kombiniert werden. So ist beispielsweise in einem bevorzugten TTS die Wirkstoffschicht frei von Tocopherolen, und das Polymer/die Polymere der Polymermatrix dieser Wirkstoffschicht enthält/enthalten weder Hydroxylgruppen noch Carboxylgruppen. Zudem befindet sich in dieser Ausführungsform zwischen der Wirkstoffschicht und der Klebeschicht bevorzugt eine die Freisetzung von Rivastigmin kontrollierende Membran, und das TTS eignet sich für einen Anwendungszeitraum von mindestens zwei, mindestens drei oder mindestens vier Tagen, z. B. für zwei bis sieben, für drei bis sechs, oder für vier bis fünf Tage.

Der Wirkstoff Rivastigmin ist in dem erfindungsgemäßen TTS hinreichend stabil. "Hinreichend stabil" bedeutet, dass die Verunreinigungen des Wirkstoffs nach einem Monat Lagerung bei 40°C und 75 % relative Luftfeuchtigkeit insgesamt nicht mehr als 1 Gew.-% , bevorzugt nicht mehr als 0,5 Gew.-% betragen, bezogen auf den Sollgehalt an

Wirkstoff in der Formulierung. Als Verunreinigungen des Wirkstoffs in der Formulierung gelten Abbauprodukte des Wirkstoffs Rivastigmin und mit dem Wirkstoff in die Formulierung eingetragene Verunreinigungen (z. B. Spuren von Zwischenprodukten aus der Herstellung des Wirkstoffs).

Die Stabilität bzw. die Menge an Verunreinigungen kann wie in Beispiel 4 beschrieben bestimmt werden. Vorzugsweise ist der Gesamtgehalt der Zersetzungsprodukte/Verunreinigungen nach drei Monaten Lagerung bei 40°C und 75 % relative Luftfeuchtigkeit weniger als 1 Gew.-%, bevorzugter weniger als 0,6 Gew.-%. Es ist auch bevorzugt, dass der Gesamtgehalt der Zersetzungsprodukte/Verunreinigungen nach sechs Monaten Lagerung bei 40°C und 75 % relative Luftfeuchtigkeit weniger als 1 Gew.-% beträgt. Es ist weiterhin bevorzugt, dass der Gesamtgehalt der Verunreinigungen nach einem Monat Lagerung bei 25°C und 60 % relative Luftfeuchtigkeit weniger als 0,25 Gew.-% beträgt. Es ist weiterhin bevorzugt, dass der Gesamtgehalt der Verunreinigungen nach drei und nach sechs Monaten Lagerung bei 25°C und 60 % relative Luftfeuchtigkeit weniger als 0,5 Gew.-% beträgt. Die Angaben von "Gew.-%" Verunreinigungen beziehen sich immer auf den Sollgehalt an Wirkstoff in der Formulierung, soweit nicht anders angegeben.

Die Anwendungsdauer eines erfindungsgemäßen TTS beträgt bevorzugt mindestens zwei oder mindestens drei Tage. In einer speziellen Ausführungsform ist das erfindungsgemäße TTS für eine Anwendungsdauer von 2 bis 4, 2 bis 5, 2 bis 6, 2 bis 7 oder 3 bis 8 Tagen geeignet.

Vorzugsweise enthält die Wirkstoffschicht, bevorzugter das gesamte TTS, kein Tocopherol. In einer weiteren Ausführungsform enthält die Wirkstoffschicht, vorzugsweise das gesamte TTS, kein Tocopherol und kein Butylhydroxdyanisol (BHT, auch Butyl-p-Kresol genannt). In einer weiteren Ausführungsform enthält die Wirkstoffschicht, vorzugsweise das gesamte TTS, kein Tocopherol, kein Butylhydroxdyanisol und kein Butylhydroxytoluol. In einer besonderen Ausführungsform enthält die Wirkstoffschicht, vorzugsweise das gesamte TTS, keines der folgenden Antioxidationsmittel: Tocopherole und dessen Ester, das Sesamol des Sesamöls, das Koniferylbenzoat des Benzoeharzes, die Nordihydroguajakharz- und - guajaretsäure (NDGA), die Gallate (Methyl-, Ethyl-, Propyl-, Amyl-, Butyl-, Lauryl- u.a. Gallate), das Butylhydroxdyanisol (BHT, auch Butyl-p-Kresol genannt); Ascorbinsäure und Salze davon, Ascorbylpalmitat, Erythorbinsäure (iso-Ascorbinsäure) und Salze davon, Monothioglycerol, Natriumformaldehydsulfoxylat, Natriummetabisulfit, Natriumbisulfit, Natriumsulfit, Kaliummetabisulfit, butyliertes Hydroxyanisol, butyliertes Hydroxytoluol, Propionsäure. In einer speziellen Ausführungsform enthält die Wirkstoffschicht, bevorzugter das gesamte TTS, überhaupt keine Antioxidationsmittel.

Es ist jedoch durchaus möglich, dass in dem TTS der vorliegenden Erfindung Antioxidationsmittel vorhanden sein können, sofern diese die Wirkungsweise des TTS nicht negativ beeinflussen. Es ist hierbei zu beachten, dass zur Stabilisierung von Rivastigmin gemäß einem Aspekt der vorliegenden Erfindung keine Antioxidationsmittel nötig sind. Jedoch könnten Antioxidationsmittel auch für andere Zwecke in den erfindungsgemäßen TTS zum Einsatz kommen. Es ist daher möglich, wenn auch nicht bevorzugt, dass das erfindungsgemäße TTS Antioxidationsmittel enthält, z. B. Tocopherole wie $\alpha$-Tocopherol und dessen Ester, Butylhydroxytoluol und Butylhydroxyanisol.

Die Gesamtmenge an Antioxidationsmittel im TTS der vorliegenden Erfindung ist üblicherweise weniger als 1 Gew.-% oder weniger als 0.1 Gew.-%, bevorzugter weniger als 0.05 Gew.-%, am bevorzugtesten weniger als 0.01 Gew.-%, jeweils bezogen auf das Gewicht der Gesamtformulierung (exklusive der Deck- und Abziehschicht).

*Aufbau des TTS*

Der Aufbau der erfindungsgemäßen TTS umfasst mehrere Schichten. Auf dem bei der Anwendung der Haut abgewandten Ende des TTS befindet sich die Deckschicht. An der der menschlichen Haut beim Gebrauch zugewandten Seite der Deckschicht befindet sich die Wirkstoffschicht. Weiterhin befindet sich an der der menschlichen Haut beim Gebrauch zugewandten Seite der Wirkstoffschicht die Klebeschicht. An der der menschlichen Haut beim Gebrauch zugewandten Seite der Klebeschicht befindet sich vor der Anwendung des TTS die Abziehschicht, welche unmittelbar vor dem Gebrauch des TTS entfernt wird. Bevorzugt befindet sich zwischen der Wirkstoffschicht und der Klebeschicht eine die Freisetzung des Wirkstoffs kontrollierende Membran.

Die Fläche des erfindungsgemäßen TTS ist nicht besonders eingeschränkt. Üblicherweise beträgt die Fläche etwa 5-40 $cm^2$, kann jedoch durchaus größer oder kleiner sein.

Die Fläche der Deckschicht des erfindungsgemäßen TTS entspricht in einer Ausführungsform mindestens der Fläche der Wirkstoffschicht beziehungsweise der Klebeschicht. Sie kann jedoch auch größer als die der Wirkstoffschicht sein, sodass sie nicht nur die Wirkstoffschicht vollständig bedeckt, sondern auch über den Rand der Wirkstoffschicht hinausragt. In einer solchen Ausführungsform sollte jedoch entweder die Fläche der Klebeschicht gleich der Fläche der Deckschicht sein oder die der Haut zugewandten Seite der Deckschicht eine weitere Klebeschicht aufweisen, um zu gewährleisten, dass die gesamte der Haut zugewandten Oberfläche des TTS bei der Anwendung auf der Haut haftet. In einer anderen Ausführungsform ist die Deckschicht etwas kleiner als die Fläche der Wirkstoffschicht und/oder Klebeschicht.

*Wirkstoffschicht*

Die Wirkstoffschicht des erfindungsgemäßen TTS enthält den Wirkstoff Rivastigmin, eingebettet in eine Polymermatrix. Die Polymermatrix umfasst gemäß der Erfindung keine Polymere oder Copolymere, die freie Hydroxylgruppen oder freie Carboxylgruppen enthalten. Vorzugsweise enthält die Polymermatrix im wesentlichen keine freien Hydroxylgruppen und keine freien Carboxylgruppen. Bevorzugter enthält die Polymermatrix im wesentlichen keine freien Aminogruppen, keine freien Hydroxylgruppen und keine freien Carboxylgruppen. Die Polymermatrix wird vorzugsweise aus Polymeren und/oder Copolymeren gebildet, die keine freien Hydroxylgruppen und keine freien Carboxylgruppen enthalten. Noch bevorzugter wird die Polymermatrix aus Polymeren und/oder Copolymeren gebildet, die keine freien Aminogruppen, keine freien Hydroxylgruppen und keine freien Carboxylgruppen enthalten.

Geeignete Polymere oder Copolymere ohne freie funktionelle Gruppen, die die Polymermatrix bilden, sind bestimmte Polyacrylate, Acrylat-Vinylacetat Copolymere, Polyisobutylen und Styrol-Butadien Copolymere, welche einzeln oder als Mischung vorliegen können.

Als geeignete Polyacrylate, die im wesentlichen keine freien funktionellen Gruppen enthalten, können Polymere (Homopolymere, Copolymere und Block-Copolymere) auf Basis von Acrylsäureestern und/oder Methacrylsäureestern verwendet werden. Als Monomere zur Herstellung geeigneter Polyacrylate kommen dabei insbesondere n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen dieser Monomere in Frage. Es handelt sich bei diesen Monomeren um Ester der Acryl- bzw. Methacrylsäure, die lineare, verzweigte oder cyclische aliphatische $C_1$-$C_{12}$- Substituenten ohne sonstige freie funktionelle Gruppen tragen. Auch Vinylacetat kann als Co-Monomer zusammen mit mindestens einem dieser Monomere zur Herstellung des Polyacrylats verwendet werden.

Die Polymermatrix besteht vorzugsweise aus einem oder mehreren Polyacrylaten, die im wesentlichen keine freien funktionellen Gruppen enthalten. Bevorzugter besteht die Polymermatrix aus Polyacrylaten, die durch Polymerisierung von Acrylsäureestern und/oder Methacrylsäureestern hergestellt wurden. In einer besonderen Ausführungsform besteht die Polymermatrix aus Polyacrylaten, die durch Polymerisierung von Acrylsäureestern und/oder Methacrylsäureestern hergestellt wurden, wobei die Acrylsäureester und/oder Methacrylsäureester ausgewählt sind aus der Gruppe bestehend aus n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen davon. In einer anderen Ausführungsform besteht die Polymermatrix im wesentlichen aus Polyacrylaten, die durch Copolymerisierung von Acrylsäureestern und/oder Methacrylsäureestern mit Vinylacetat hergestellt wurden, wobei die Acrylsäureester und/oder Methacrylsäureester ausgewählt sind aus der Gruppe bestehend aus n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen davon.

Besonders bevorzugt sind Copolymere, die aus den Startmonomeren 2-Ethylhexylacrylat und Vinylacetat hergestellt werden, z. B. ein Acrylat-Vinylacetat Copolymer, welches zu jeweils 50 % aus den Startmonomeren 2-Ethylhexylacrylat und Vinylacetat hergestellt wird (Duro-Tak® 87-4098). Ebenfalls bevorzugt ist das Acrylat-Polymer Duro-Tak® 87-9088 (ebenfalls ein Acrylat-Polymer ohne freie funktionelle Gruppen), erhältlich von der Firma Henkel. In einer speziellen Ausführungsform wird für die Polymermatrix das Acrylat-Polymer Duro-Tak® 87-900A oder Duro-Tak® 87-9301 eingesetzt.

Der Gesamtanteil von Monomeren, die freie Hydroxylgruppen oder freie Carboxylgruppen enthalten (z. B. Acrylsäure, Methacrylsäure und Ester der Acrylsäure bzw. Methacrylsäure, welche funktionelle Gruppen tragen, insbesondere die hydroxylgruppenhaltigen Ester), liegt unterhalb von 1 Gew. -%, vorzugsweise unterhalb von 0,5 Gew. -%, bevorzugter unterhalb von 0,2 ,Gew.-%; bezogen auf das Monomerengemisch, aus der die Polymermatrix hergestellt wird. In einer besonderen Ausführungsform liegt der Gesamtanteil dieser Monomere unterhalb von 0,1 Gew. -%. In einer besonderen Ausführungsform sind keine freien Hydroxylgruppen und keine freien Carboxylgruppen im Monomerengemisch enthalten.

Ein TTS, welches als wirkstoffhaltige Polymermatrix Polyacrylate enthält, die im wesentlichen frei von Hydroxylgruppen und Carboxylgruppen sind, wurde zwar in der WO 03/017988 A1 bereits beschrieben, jedoch nicht in Verbindung mit dem Wirkstoff Rivastigmin. Die in der WO 03/017988 beschriebene Aufgabe war es, den Nachteil der geringen Wirkstoffausnutzung eines TTS zu lösen. Gemäß dieser Offenbarung wurde diese Aufgabe durch Polymermatrices gelöst, welche im Idealfall frei von Hydroxylgruppen oder Carboxylgruppen sind. Der Wirkstoff Rivastigmin wird in dieser Druckschrift nicht erwähnt, geschweige denn eine die Stabilität von Rivastigmin steigernde Wirkung beschrieben.

Die Wirkstoffschicht enthält gemäß der Erfindung keine Polymeren oder Copolymeren, die freie Hydroxylgruppen oder freie Carboxylgruppen enthalten. Weiterhin enthält die Wirkstoffschicht keine freien Hydroxylgruppen und

keine freien Carboxylgruppen. Bevorzugter enthält die Wirkstoffschicht im wesentlichen keine freien Aminogruppen, keine freien Hydroxylgruppen und keine freien Carboxylgruppen. In einer besonderen Ausführungsform enthält auch die Klebeschicht im wesentlichen keine Polymeren oder Copolymeren, die freie Hydroxylgruppen oder freie Carboxylgruppen enthalten. Vorzugsweise enthält die Klebeschicht im wesentlichen keine freien Hydroxylgruppen und keine freien Carboxylgruppen. Bevorzugter enthält die Klebeschicht im wesentlichen keine freien Aminogruppen, keine freien Hydroxylgruppen und keine freien Carboxylgruppen.

Die Wirkstoffschicht enthält vorzugsweise 30 - 50 Gew.-% Rivastigmin und 50 - 70 Gew.-% Polymermatrix, bezogen auf das Gesamtgewicht der Wirkstoffschicht. In einer besonders bevorzugten Ausführungsform des erfindungsgemäßen TTS enthält die Wirkstoffschicht etwa 40 Gew.-% Rivastigmin und etwa 60 Gew.-% Polymermatrix. Bevorzugt enthält die Wirkstoffschicht neben dem Wirkstoff und der Polymermatrix keine weiteren Bestandteile. Es ist jedoch möglich, dass zusätzlich weitere im Stand der Technik bekannte Zusatzstoffe in der Wirkstoffschicht enthalten sind. So können beispielsweise Weichmacher oder Gelbildner zusätzlich in der Wirkstoffschicht vorhanden sein.

Die absolute Menge an Rivastigmin hängt von verschiedenen Faktoren, insbesondere von der Größe des einzusetzenden TTS, dem Flächengewicht und der Wirkstoffkonzentration in der Wirkstoffschicht ab. Die Flächengewichte der getrockneten Wirkstoffschicht-Matrix liegen bevorzugt in einem Bereich von 20 - 100 g/m$^2$, weiter bevorzugt in einem Bereich von 25 - 80 g/m$^2$ und noch weiter bevorzugt in einem Bereich von 30 - 70 g/m$^2$. Die Wirkstoffschicht kann eine Dicke (Trockendicke) im Bereich von 20 - 400 μm, oder 30 - 200 μm oder 40 - 100 μm aufweisen. Auch andere Dicken als die vorstehend genannten sind möglich.

*Zwischenmembran*

Die gemäß der vorliegenden Erfindung zwischen Wirkstoffschicht - und Klebeschicht liegende, die Freisetzung des Wirkstoffs kontrollierende Membran (auch als "Kontrollmembran" bezeichnet) besteht bevorzugt aus einem Polyolefin, wie beispielsweise Polypropylen (z.B. Celgard® 2400) oder besonders bevorzugt aus Polyethylen (z.B. CoTran™ 9719 oder CoTran™ 9720) oder weiter bevorzugt aus Polyethylen mit einem Vinylacetat-Anteil von 4,5 bis 19 % (z.B. CoTran™ 9707, CoTran™ 9702, CoTran™ 9728). Desweiteren können die Membranen eine Porosität von bis zu 90 % aufweisen (z.B. Solupor® 10P05A, Celgard® 2400).

Es können poröse Membranen oder kohärente Membranen eingesetzt werden.

Die Porosität der porösen Membranen kann bis zu etwa 90% betragen. Daten bevorzugter poröser Membranen sind:

Solupor® 10P05A (Polyethylen, Porosität: 83%, Dicke: 60 μm)
Celgard® 2400 (Polypropylen, Porosität: 41%, Dicke: 25 μm)

Daten bevorzugter kohärenter Membranen sind:

CoTran™ 9719 (Polyethylen, Dicke: 43,2 μm)
CoTran™ 9720 (Polyethylen, Dicke: 76,2 μm)
CoTran™ 9707 (Polyethylen mit 4,5% Vinylacetat, Dicke: 50,8 μm)
CoTran™ 9702 (Polyethylen mit 9,0% Vinylacetat, Dicke: 50,8 μm)
CoTran™ 9728 (Polyethylen mit 19,0% Vinylacetat, Dicke: 50,8 μm)

Die Membran ist üblicherweise zwischen 0,01 und 0,15 mm dick. Die bevorzugte Dicke der Membran beträgt 0,025 bis 0,080 mm.

Bevorzugt wird erfindungsgemäß eine kohärente Membran, bestehend im wesentlichen aus Polyethylen, mit einer Dicke von etwa 40 bis 80 μm (z. B. CoTran™ 9720) eingesetzt. Ebenfalls bevorzugt wird erfindungsgemäß eine kohärente Membran, bestehend im wesentlichen aus Polyethylen mit etwa 19,0% Vinylacetat (z. B. CoTran™ 9728) eingesetzt. Am bevorzugtesten wird erfindungsgemäß eine kohärente Membran, bestehend im wesentlichen aus Polyethylen, mit einer Dicke von etwa 40 bis 50 μm (z. B. CoTran™ 9719) eingesetzt.

Die Membran ist der essentielle Formulierungsbestandteil zur Gewährleistung einer kontrollierten mehrtägigen Abgabe des Wirkstoffs. Die Arzneistoffabgabe kann unter anderem durch den Vinylacetat-Anteil in der Membran gesteuert werden.

*Klebeschicht*

Die Klebeschicht des erfindungsgemäßen TTS enthält vorzugsweise Polyisobutylen als Haftkleber und gegebenenfalls Polybuten um die Klebkraft zu verbessern. Polyisobutylen ist ein selbstklebender Haftkleber, der nicht aushärtet und daher seine klebenden Eigenschaften über einen langen Zeitraum beibehält. Bevorzugterweise werden

Polyisobutylene mit unterschiedlichem mittlerem Molekulargewicht als Mischung eingesetzt. Polyisobutylen ist in verschiedenen mittleren Molekulargewichten erhältlich. Der Ausdruck "mittleres Molekulargewicht" im Zusammenhang mit Polyisobutylen bezieht sich in der vorliegenden Anmeldung auf das sog. Viskositätsmittel Mv. Das Viskositätsmittel Mv wird aus der Lösungsviskosität einer Lösung des Polyisobutylens in Isooctan bei 20 °C bestimmt. Als Meßgerät dient ein Ubbelohde-Viskosimeter. Das Viskositätsmittel Mv errechnet sich aus folgender Formel:

$$M_v = \frac{0.65}{} \sqrt{\frac{J_o \times 10^2}{3.06}}$$

Die Ermittlung des zur Bestimmung des Viskositätsmittels $M_V$ benötigten Staudingerindex $J_o$ erfolgt nach der Schulz-Blaschke-Beziehung aus der gemessenen spezifischen Viskosität $\eta_{SP}$ und der Lösungskonzentration.

$$J_o \quad = \eta_{sp}/c\ (1 + 0.31 \times \eta_{sp})\ cm^3/g$$

(Schulze-Blaschke-Beziehung)

Die spezifische Viskosität $\eta_{SP} = t/t_0 - 1$, wobei t und $t_0$ die Flusszeit der Lösung bzw. des Lösungsmittels (jeweils mit Hagenbach-Couette-Korrektur) sind, und c die Konzentration der Lösung in $g/cm^3$ ist. Gegebenenfalls kann die Vorschrift DIN 53728 ergänzend herangezogen werden.

Geeignete mittlere Molekulargewichte $M_V$ von Polyisobutylen liegen beispielsweise im Bereich von etwa 40.000 g/mol bis etwa 4.000.000 g/mol. Eine mögliche Mischung ist die von (1) Polyisobutylen mit einem mittleren Molekulargewicht Mv von etwa 40.000 g/mol (z. B. Oppanol® B10, erhältlich von der Firma BASF) und (2) Polyisobutylen mit einem mittleren Molekulargewicht Mv von über etwa 1.000.000 g/mol (z. B. Oppanol® B100, erhältlich von der Firma BASF, mit einem mittleren Molekulargewicht Mv von etwa 1.110.000 g/mol). Es liegt innerhalb der Kenntnis eines Fachmanns, die verschiedenen Molekulargewichte im geeigneten Verhältnis zu mischen, um die gewünschten Eigenschaften der Klebeschicht zu erzielen.

Das Polyisobutylen in der Klebeschicht kann eine Molekulargewichtsverteilung aufweisen, die ein erstes relatives Maximum zwischen 30.000 g/mol und 100.000 g/mol, und ein zweites relatives Maximum zwischen 300.000 g/mol und 500.000 g/mol aufweist. Bevorzugter liegt das erste relative Maximum zwischen 35.000 g/mol und 50.000 g/mol und, unabhängig davon, dass zweite relative Maximum zwischen 350.000 g/mol und 450.000 g/mol. Am bevorzugtesten liegt das erste relative Maximum bei etwa 40.000 g/mol, und, unabhängig davon, das zweite relative Maximum bei etwa 400.000 g/mol.

Das Polyisobutylengemisch des Haftklebers kann dadurch erhalten werden, dass man ein erstes Polyisobutylenpolymer mit einem mittleren Molekulargewicht $M_V$ zwischen 30.000 g/mol und 100.000 g/mol mit einem zweiten Polyisobutylenpolymer mit einem mittleren Molekulargewicht $M_V$ zwischen 300.000 g/mol und 500.000 g/mol mischt. Das erste Polyisobutylenpolymer hat vorzugsweise ein mittleres Molekulargewicht $M_V$ zwischen 35.000 g/mol und 50.000 g/mol, am bevorzugtesten von etwa 40.000 g/mol. Das zweite Polyisobutylenpolymer hat vorzugsweise ein mittleres Molekulargewicht $M_V$ zwischen 350.000 g/mol und 450.000 g/mol, am bevorzugtesten von etwa 400.000 g/mol.

Die bevorzugteste Mischung ist die von (1) Polyisobutylen mit einem mittleren Molekulargewicht $M_V$ von etwa 40.000 g/mol (zum Beispiel Oppanol® B10 SFN, erhältlich von der Firma BASF) und (2) Polyisobutylen mit einem mittleren Molekulargewicht Mv von etwa 400.000 g/mol (zum Beispiel Oppanol® B50 SF, erhältlich von der Firma BASF).

Die Anteile der beiden Polyisobutylenpolymere in dem Gemisch können variieren. Das Gewichtsverhältnis des ersten Polyisobutylenpolymer zum zweiten Polyisobutylenpolymer in dem Gemisch kann 10:1 bis 1:10 betragen, vorzugsweise 2:1 bis 1:2, am bevorzugtesten 3:2 bis 2:3. In besonders bevorzugten Ausführungsformen besteht das Polyisobutylenpolymer des Haftklebers aus 40 bis 60 Gew.-% Oppanol® B10 (zum Beispiel Oppanol® B10 SFN) und 60 bis 40 Gew.-% Oppanol® B50SF.

Vorzugsweise enthält die erfindungsgemäß verbesserte Klebeschicht zusätzlich Polybuten. Geeignete mittlere Molekulargewichte $M_N$ von Polybuten liegen beispielsweise im Bereich von etwa 500 bis 10.000 g/mol. Auch Polybuten liegt bevorzugt in einer Mischung von unterschiedlichen durchschnittlichen Molekulargewichten vor. Eine bevorzugte Mischung ist die von (1) Polybuten mit einem mittleren Molekulargewicht $M_N$ von etwa 900 g/mol (z.B. Indopol® H-100, mit einem mittleren Molekulargewicht $M_N$ = 910 g/mol, erhältlich von der Firma Ineos) und (2) Polybuten mit einem mittleren Molekulargewicht $M_N$ von etwa 6.000 g/mol (z.B. Indopol® H-18000, erhältlich von der Firma Ineos).

Eine andere bevorzugte Mischung ist die von (1) Polybuten mit einem mittleren Molekulargewicht $M_N$ von etwa 900 g/mol (z.B. Indopol® H-100, mit einem mittleren Molekulargewicht $M_N$ = 910 g/mol, erhältlich von der Firma Ineos) und (2) Polybuten mit einem mittleren Molekulargewicht $M_N$ von etwa 2.500 g/mol (z.B. Indopol® H-1900, erhältlich von

der Firma Ineos).

Besonders bevorzugt ist die Mischung von (1) Polybuten mit einem mittleren Molekulargewicht $M_N$ von etwa 2.500 (z.B. Indopol® H-1900, erhältlich von der Firma Ineos) und (2) Polybuten mit einem mittleren Molekulargewicht $M_N$ von etwa 6.000 g/mol (z.B. Indopol® H-18000). Es liegt innerhalb der Kenntnis eines Fachmanns, die verschiedenen Molekulargewichte im geeigneten Verhältnis zu mischen, um im System die gewünschten Eigenschaften der Klebeschicht hinsichtlich Klebkraft und Freisetzungsrate des Wirkstoffs zu erzielen.

Die Anteile zweier Polybutene in der Klebeschicht können variieren. Das Gewichtsverhältnis des ersten Polybutens zum zweiten Polybuten beträgt bevorzugt 2:1 bis 1:2, am bevorzugtesten 3:2 bis 2:3. In einer besonders bevorzugten Ausführungsform besteht das Polybutenpolymer in der Klebeschicht aus ewas 40 Gew.-% Polybuten mit einem mittleren Molekulargewicht $M_N$ von etwa 2.500 g/mol und aus etwa 60 Gew.-% Polybuten mit einem mittleren Molekulargewicht $M_N$ von 6.000 g/mol.

Das "mittlere Molekulargewicht $M_N$" ist das Zahlenmittel der Molmasse und kann bestimmt werden gemäß "American Standard" ASTM D3536-91 oder ASTM D5296-05.

Die erfindungsgemäßen Bestandteile der verbesserten Klebeschicht Polyisobutylen und Polybuten liegen bevorzugt im Gewichtsverhältnis von 4:1 bis 1:2, bevorzugter 3:1 bis 1:2 vor. Beispielsweise können Polyisobutylen und Polybuten im Gewichtsverhältnis von etwa 1:1 vorliegen. In einer anderen Ausführungsform liegen Polyisobutylen und Polybuten im Gewichtsverhältnis von etwa 7:3 vor.

Die Klebeschicht enthält bevorzugt etwa 10 bis etwa 90 Gew.-%, vorzugsweise etwa 25 bis etwa 75 Gew.-%, am bevorzugtesten etwa 40 bis etwa 70 Gew.-% Polyisobutylen (z. B. ewa 50 Gew.-% oder etwa 70 Gew.-%), bezogen auf das Gesamtgewicht der Klebeschicht. Diese Anteile bezeichnen den Gesamtgehalt an Polyisobutylen bzw. PolyisobutylenGemisch in der Klebeschicht. Die Klebeschicht enthält ebenfalls bevorzugt etwa 5 bis ewa 80 Gew.-%, vorzugsweise etwa 15 bis etwa 60 Gew.-%, am bevorzugtesten etwa 25 bis etwa 50 Gew.-% Polybuten (z. B. etwa 50 Gew.-% oder etwa 30 Gew.-%), bezogen auf das Gesamtgewicht der Klebeschicht. Diese Anteile bezeichnen den Gesamtgehalt an Polybuten bzw. Polybuten -Gemisch in der Klebeschicht.

In einer bevorzugten Ausführungsform enthält die Klebeschicht kein Acrylatpolymer und kein Acrylat-Copolymer.

Die Dicke der Klebeschicht (Trockendicke) ist nicht besonders eingeschränkt. Sie kann in einem Bereich von etwa 10 - 300 μm, oder in einem Bereich von 70 -140 μm liegen. Die absolute Menge der Klebeschicht kann etwa 10 - 50 $g/m^2$, oder 20 - 40 $g/m^2$ betragen, ohne darauf eingeschränkt zu sein.

Die Klebeschicht enthält im allgemeinen 60 - 100 Gew.-% des Haftklebers (z. B. Polyisobutylen) bzw. des Haftklebergemisches einschließlich Klebkraftverstärker (z.B. Polybuten). Weitere mögliche Bestandteile der Klebeschicht sind Weichmacher und Gelbildner.

Geeignete Weichmacher sind im Stand der Technik bekannt, wobei es sich bevorzugt um Mineralöl, Neutralöl, Paraffin, Leinsamenöl, Octylpalmitat, Squalen, Squalan, Silikonöl, Isobutylmyristat, Isostearylalkohol und/oder Oleylalkohol, besonders bevorzugt um Mineralöl, Neutralöl und/oder Paraffin handelt. Mineralöle sind farblose, klare Kohlenwasserstoffe. Sie werden aus den oberhalb von etwa 300°C siedenden Destillationsfraktionen des Erdöls gewonnen und durch Abkühlen von festen Kohlenwasserstoffen befreit. Durch geeignete Fraktionierung können Mineralöle gewonnen werden, die etwa bei Körpertemperatur, also bei etwa 35 - 37°C flüssig sind und bei tiefen Temperaturen, insbesondere bei Temperaturen unter 20°C fest sind. Die Auswahl des Mineralöls mit einem Verflüssigungspunkt von etwa 30 - 35°C ist bevorzugt. Besonders bevorzugt sind Paraffine und Mineralöle, welche den Anforderungen der Ph. Eur. 6 und/oder der USP 32-NF 27 entsprechen.

Der Weichmacher ist in der Klebeschicht im allgemeinen in einer Menge im Bereich von 0 - 40 Gew.-%, oder von 1 - 10 Gew.-%, oder im Bereich von 2 - 5 Gew.-%, beispielsweise 2 Gew.-% bezogen auf das Gesamtgewicht der Klebeschicht, vorhanden.

Bei dem Gelbildner handelt es sich bevorzugt um einen Gelbildner mit partikulärer Struktur, welcher auf seiner Oberfläche eine hohe Konzentration polarer Gruppen aufweist. Diese verursachen zu den Ölen hin entsprechend hohe Grenzflächenspannungen, die durch Agglomeration der Partikel untereinander zu Gelgerüsten teilweise kompensiert werden. Demzufolge sind die Gelgerüste immer umso fester, je größer der Polaritätsunterschied zwischen den Ölen und der Gerüstbildneroberfläche ist. Bevorzugt wird erfindungsgemäß als Gelbildner hochdisperses Siliziumdioxid oder pyrogene Kieselsäure eingesetzt. Die Größe der Partikel bewegt sich bevorzugt im Nanometerbereich und liegt zum Beispiel im Bereich von 400 - 1500 nm, insbesondere im Bereich von 500 - 1000 nm. Pyrogene Kieselsäure wird beispielsweise unter der Bezeichnung CAB-O-SIL® vertrieben und ist ein bekanntes Verdickungsmittel für Mineralöl. Ein anderes Beispiel für einen geeigneten Gelbildner ist Bentonit. Auch das als Gelbildner bekannte Natriumcarbomer kann verwendet werden.

Eingesetzt wird der Gelbildner bevorzugt in einer Menge von 0 - 4,0 Gew.-%, weiter bevorzugt 0,1 - 2,0 Gew.-%, noch weiter bevorzugt 0,5 - 2,0 Gew.-%, bezogen auf das Gewicht der Klebeschicht.

In einer besonders bevorzugten Ausführungsform der erfindungsgemäßen TTS besteht die Klebeschicht zu 99,5 Gew.-% aus Mischungen von Polyisobutylen mit unterschiedlichen Molekulargewichten und Polybuten mit unterschiedlichen Molekulargewichten und zu 0,5 Gew.-% Gelbildner, bevorzugterweise hochdispersem Siliziumdioxid oder pyrogener Kieselsäure, bezogen auf das Gesamtgewicht der Klebeschicht.

*Deckschicht*

Die Deckschicht der erfindungsgemäßen TTS ist vorzugsweise okklusiv, also abschließend. Derartige Deckschichten können in bevorzugter Ausführungsform aus Polyolefinen, insbesondere Polyethylen, oder aus Polyester sowie Polyurethanen bestehen. Auch Schichten, die mehrere verschiedene Polymere übereinander angeordnet beinhalten, sind vorteilhafterweise einsetzbar. Geeignete Materialien umfassen Polyolefin, Zellophan, Celluloseacetat, Ethylcellulose, mit Weichmachern versehene Vinylacetat-Vinylchlorid Copolymere, Ethylen-Vinylacet Copolymere, Polyethylentherephtalat, Nylon, Polyethylen, Polypropylen, Polyvinylidenchlorid, Ethylen-Metacrylat Copolymere, Papier, das gegebenenfalls beschichtet sein kann, textile Gewebe, Aluminiumfolie und Polymer-Metall Kompositmaterialien. Besonders bevorzugt sind Polyesterfolien, wie Polyethylentherephtalatfolien. Die Dicke der Rückschicht kann, wie im Stand der Technik üblich, zum Beispiel 10 μm bis 100 μm, zum Beispiel etwa 40 μm (nominelle Dicke) betragen. Ganz besonders bevorzugt sind Verbundfolien aus pigmentiertem PE, PETP und Aluminium.

*Abziehschicht*

Auf der Klebeschicht befindet sich erfindungsgemäß eine Abziehschicht, auch als "release liner" bezeichnet. Diese Abziehschicht ist bevorzugt aus polymerem Material hergestellt, das gegebenenfalls auch metallisiert sein kann. Beispiele für bevorzugt eingesetzte Materialien sind Polyurethane, Polyvinylacetat, Polyvinylidenchlorid, Polypropylen, Polycarbonat, Polystyrol, Polyethylen, Polyethylentherephtalat, Polybutylentherephtalat sowie gegebenenfalls mit entsprechenden Polymeren oberflächenbeschichtetes Papier. Bevorzugt handelt es sich hierbei um eine einseitig oder beidseitig fluoropolymerbeschichtete oder silikonisierte Abziehschicht. Besonders bevorzugt sind handelsübliche fluoropolymerbeschichtete oder silikonisierte Polyesterfolien, wie die einseitig silikonisierten Handelsprodukte Primeliner 100 μm und Perlasic LF 75 μm (Firma Loparex, NL und Perlen Converting AG, Schweiz) oder die einseitig fluoropolymerbeschichtete Produkte wie z.B. ScotchPak 1022 (3M Drug delivery).

*Weitere Aspekte und Ausführungsformen*

Eine besonders bevorzugte Ausführungsform der erfindungsgemäßen TTS ist ein TTS zur Verabreichung eines Wirkstoffes durch die Haut, umfassend:

a) eine Deckschicht,

b) ein auf der Deckschicht befindliche Wirkstoffschicht enthaltend 30 - 50 Gew.-% Wirkstoff und 50 - 70 % Polymermatrix, bezogen auf das Gesamtgewicht der Wirkstoffschicht, wobei die Polymermatrix im wesentlichen aus einem Acrylat-Polymer ohne Hydroxylgruppen und ohne Carboxylgruppen, oder im wesentlichen aus einem Acrylat-Copolymer ohne Hydroxylgruppen und ohne Carboxylgruppen besteht, und wobei der Wirkstoff Rivastigmin oder ein physiologisch verträgliches Salz, Hydrat, Solvat oder Derivat davon ist;

c) eine auf der Wirkstoffschicht befindliche, die Freisetzung des Rivastigmins kontrollierende Membran;

d) eine auf der Membran befindliche Klebeschicht bestehend aus 0 - 1 Gew.-% Siliziumdioxid und 99 - 100 Gew.-% eines Gemisches aus einem Polyisobutylenpolymer mit einem mittleren Molekulargewicht Mv von etwa 40.000 g/mol, einem Polyisobutylenpolymer mit einem mittleren Molekulargewicht $M_V$ von etwa 400.000 g/mol, einem Polybutenpolymer mit einem mittleren Molekulargewicht $M_N$ von etwa 2.500 g/mol und einem Polybutenpolymer mit einem mittleren Molekulargewicht $M_N$ von etwa 6.000 g/mol; und

e) eine auf der Klebeschicht befindliche Abziehschicht,
wobei das Polymer/die Polymere der Polymermatrix keine freien Hydroxylgruppen und keine freien Carboxylgruppen enthält/enthalten und die Wirkstoffschicht keine freien Hydroxylgruppen und keine freien Carboxylgruppen enthält.

Besonders bevorzugt ist die Menge an Antioxidationsmittel in der Gesamtformulierung dieser Ausführungsform ohne Deck- und Abziehschicht weniger als 0,1 Gew.-%, bevorzugt weniger als 0,01%.

In einer besonderen Ausführungsform enthält das erfindungsgemäße TTS kein Antioxidationsmittel ausgewählt aus der Gruppe bestehend aus Vitamin E und Estern davon, Butyhydroxytoluol und Butylhydroxyanisol.

Vorzugsweise weist das erfindungsgemäße TTS ein im wesentlichen lineares Permeationsprofil auf. Die Permeation ist dabei im wesentlichen linear über einen Zeitraum von wenigstens 24 Stunden, vorzugsweise wenigstens 48 Stunden, bevorzugter wenigstens 72 Stunden, am bevorzugtesten wenigstens 96 Stunden, oder wenigstens 120 Stunden, oder wenigstens 144 Stunden, oder wenigstens 168 Stunden. Die Permeation kann durch dem Fachmann an sich bekannte Permeationstests bestimmt werden, beispielsweise durch den in vitro-Permeationstest gemäß "OECD GUIDELINE FOR THE TESTING OF CHEMICALS. Skin Absorption: in vitro method." Test Guideline 428. Adopted 13 April 2004: 1-8. Der Ausdruck "im wesentlichen linear" bedeutet vorzugsweise, dass sich die Permeationssrate, ausgedrückt in permeierter Menge pro Zeiteinheit und Flächeneinheit über ein längeres Zeitintervall nicht signifikant ändert, so dass das Permeationsprofil im wesentlichen einen linearen Verlauf aufweist. Insbesondere in der initialen Phase kann aber eine sogenannte Latenzzeit auftreten, bei der die Permeation noch nicht die konstante Permeationsrate aufweist, und ferner kann sich die Permeationsrate zu sehr späten Zeitpunkten aufgrund einer sehr starken Entleerung des Systems verringern.

In einer besonderen Ausführungsform ist die durchschnittliche Freisetzungsrate in vivo zwischen 0,1 mg/d/cm$^2$ und 2 mg/d/cm$^2$, bevorzugt zwischen 0,2 mg/d/cm$^2$ und 1 mg/d/cm$^2$ und höchst bevorzugt mind. 0,3 mg/d/cm$^2$.

Ein weiterer Aspekt der vorliegenden Erfindung ist die Bereitstellung eines Verfahrens zur Herstellung des erfindungsgemäßen TTS. Das Verfahren umfasst

i) die Herstellung einer die Wirkstoffschicht enthaltenden Komponente, enthaltend die Deckschicht und die Wirkstoffschicht, welches sich auf der Seite der Deckschicht befindet, welche als die der Haut zugewandte Seite bestimmt ist;

ii) die Herstellung einer die Klebeschicht enthaltenden Komponente, enthaltend die Abziehschicht und die sich auf der Abziehschicht befindliche Klebeschicht und gegebenenfalls eine die Freisetzung des Wirkstoffs kontrollierenden Membran;

iii) das aufeinander Laminieren der Komponenten aus i) und ii), so dass Deck- und Abziehschicht im Querschnitt des fertigen TTS die gegenüberliegenden, äußersten Schichten darstellen.

Eine Ausführungsform des Verfahrens umfasst:

i) das Auftragen und gegebenenfalls anschließende Trocknen eines Films einer die Wirkstoffschicht bildenden Zusammensetzung, gegebenenfalls in Form einer Lösung oder Dispersion in einem geeigneten Medium, auf die Seite der Deckschicht, welche als die der Haut zugewandte Seite bestimmt ist, und gegebenenfalls das Kaschieren mit der die Freisetzung des Wirkstoffs kontrollierenden Membran;

ii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und

iii) das aufeinander Laminieren der Komponenten aus i) und ii), so dass Deck- und Abziehschicht im Querschnitt des fertigen TTS die gegenüberliegenden, äußersten Schichten darstellen.

Eine andere Ausführungsform des Verfahrens umfasst:

i) das Auftragen und gegebenenfalls anschließende Trocknen eines Films einer die Wirkstoffschicht bildenden Zusammensetzung, gegebenenfalls in Form einer Lösung oder Dispersion in einem geeigneten Medium, auf eine silikonisierte Abziehfolie ("intermediate Liner") und das Kaschieren mit der Deckschicht;

ii) Abziehen der silikonisierten Abziehfolie ("intermediate Liner") und Zukaschieren der die Freisetzung des Wirkstoffs kontrollierenden Membran;

iii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und

iv) das aufeinander Laminieren der Komponenten aus ii) und iii), so dass Deck- und Abziehschicht im Querschnitt des fertigen TTS die gegenüberliegenden, äußersten Schichten darstellen.

Die Herstellung eines bevorzugten TTS kann erfolgen, indem man zunächst die Komponenten für die Wirkstoffschicht, also den Wirkstoff Rivastigmin und das matrixbildende Polymer, bzw. Copolymer oder eine Mischung davon, in einem organischen Lösungsmittel wie Heptan oder Ethylacetat dispergiert, bzw. löst (sofern das Polymer nicht bereits gelöst vorliegt). Üblicherweise liegt das matrixbildende Polymer, bzw. Copolymer oder die Mischung davon bereits in einem Lösungsmittel vor. Dabei wird erfindungsgemäß ein Polymer und/oder Copolymer eingesetzt, das wie oben im Zusammenhang mit den erfindungsgemäßen TTS definiert ist, also ein Polymer und/oder Copolymer ohne Hydroxylgruppen und ohne Carboxylgruppen. Die oben als bevorzugt genannten Ausführungsformen der Polymermatrix gelten entsprechend für das erfindungsgemäße Verfahren. Bei der Herstellung der Wirkstoffschicht wird bevorzugt ein flüchtiges organisches Lösungsmittel eingesetzt. Diese Mischung wird dann als gleichmäßige Schicht auf der Deckschicht aufgebracht und getrocknet. Sofern eine die Freisetzung des Wirkstoffs kontrollierende Membran aufgebracht werden soll, kann diese, nachdem die Wirkstoffschichtgetrocknet ist, auf der der Deckschicht gegenüberliegenden Seite der Wirkstoffschicht aufgebracht werden, anstelle des "intermediate liners". Vorzugsweise wird die Komponente für die Wirkstoffschicht zum Schutz mit einer Folie, bevorzugt eine silikonisierte Polyesterfolie, auch als "intermediate liner" bezeichnet, versehen, welche auf der der Deckschicht gegenüberliegenden Seite der Wirkstoffschicht aufgebracht wird. Alternativ, bzw. hierzu äquivalent kann die Mischung auch zunächst auf den "intermediate liner" aufgetragen und getrocknet werden, wobei die Deckschicht im Anschluss auf die dem "intermediate liner" gegenüberliegenden Seite die Wirkstoffschicht aufgebracht wird. Der "intermediate liner" wird entfernt, kurz bevor die Wirkstoffschichtkomponente mit der die Klebeschicht enthaltenden Komponente zusammengefügt wird. Gegebenenfalls kann nach Abziehen des "intermediate liners" auch die Kontrollmembran zukaschiert werden, bevor die Zusammenfügung mit der Klebeschicht erfolgt.

In einem separaten Arbeitsschritt wird die Klebeschicht hergestellt, indem das den Haftkleber bildende (in organischem Lösungsmittel gelöste) Polymerengemisch, bevorzugt Polyisobutylen von unterschiedlichem mittleren Molekulargewicht, gegebenenfalls zusammen mit dem Klebkraftverstärker, Gelbildner und/oder dem Weichmacher in einem organischen Lösungsmittel wie Heptan dispergiert wird. Bevorzugt werden jedoch der Haftkleber und der Klebkraftverstärker und/oder Weichmacher in dem organischen Lösungsmittel gelöst und anschließend der Gelbildner in dieser Lösung dispergiert. Diese Mischung wird dann auf die Abziehfolie aufgebracht und trocknen gelassen. Die oben als bevorzugt genannten Ausführungsformen der Klebeschicht gelten entsprechend für das erfindungsgemäße Verfahren.

Die in den beiden Verfahrensschritten erhaltenen Komponenten werden anschließend miteinander laminiert, und zwar bevorzugt so, dass die Klebeschicht direkt auf die Wirkstoffschicht aufgebracht wird. In denjenigen Ausführungsformen, in denen eine Membran eingesetzt wird, wird die Klebeschicht auf die Membran aufgebracht. Anschließend können aus der fertigen laminierten Folie Stücke gewünschter Größe ausgestanzt und verpackt werden.

Bei den einzelnen Verfahrensschritten werden die organischen Lösungsmittel, die erforderlich sind, um die jeweiligen Komponenten in Lösung zu bringen bzw. zu dispergieren, dadurch entfernt, dass die Produkte ansteigenden Temperaturen, gegebenenfalls auch unter Anwendung eines Unterdrucks, ausgesetzt werden.

Einen weiteren Aspekt der vorliegenden Erfindung stellt die Verwendung eines Polymers oder Copolymers, das weder Aminogruppen noch Hydroxylgruppen noch Carboxylgruppen aufweist, in einem TTS enthaltend Rivastigmin, welches für einen Anwendungszeitraum von mindestens zwei oder mindestens drei Tagen (z. B. zwei, drei, vier, fünf, sechs oder sieben Tage) geeignet ist, dar. Bevorzugt ist die Verwendung von Polyacrylaten, Acrylat-Vinylacetat Copolymeren, Polyisobutylen und Styrol-Butadien Copolymeren wie oben definiert. Erfindungsgemäß stellen diese Polymere oder Copolymere die Polymermatrix der Wirkstoffschicht dar, in die der Wirkstoff Rivastigmin eingebettet ist.

Ein weiterer Aspekt der Erfindung ist die Verwendung eines Polymers oder Copolymers, das keine freien Hydroxylgruppen und keine freien Carboxylgruppen aufweist, zur Stabilisierung von Rivastigmin in einem TTS, oder zur Verringerung des Abbaus von Rivastigmin in einem TTS. Ein weiterer Aspekt der Erfindung ist die Verwendung eines Polymers oder Copolymers, das weder freie Aminogruppen noch freie Hydroxylgruppen noch freie Carboxylgruppen aufweist, zur Stabilisierung von Rivastigmin in einem TTS, oder zur Verringerung des Abbaus von Rivastigmin in einem TTS.

In der erfindungsgemäßen Verwendung kommen bevorzugt aminogruppenfreie, hydroxylgruppenfreie und carboxylgruppenfreie Polyacrylate und Polyacrylatcopolymere, wie Acrylat-Vinylacetat Copolymere zum Einsatz.

In einer besonders bevorzugten Ausführungsform der erfindungsgemäßen Verwendung wird das Acrylat-Vinylacetat Copolymer Duro-Tak® 87-4098 verwendet.

In einer weiteren, besonders bevorzugten Ausführungsform der erfindungsgemäßen Verwendung wird das Acrylat- polymer Duro-Tak® 87-9088 verwendet.

Einen weiteren Aspekt der vorliegenden Erfindung stellt die Verwendung von Polyisobutylen und Polybuten als ausschließliche Bestandteile des Haftklebers/Klebkraftverbesserers in der Klebeschicht eines TTS dar, welches für einen Anwendungszeitraum von mindestens zwei oder mindestens drei Tagen geeignet ist. Polyisobutylen und Polybuten liegen dabei gemäß den obenstehenden Aspekten der vorliegenden Erfindung bevorzugt jeweils als Mischungen unterschiedlicher mittlerer Molekulargewichte vor.

Ein weiterer Aspekt der vorliegenden Erfindung ist die Bereitstellung der erfindungsgemäßen TTS zur Behandlung von Alzheimer und Parkinsondemenz. Das erfindungsgemäße TTS wird dabei bevorzugt für einen Anwendungszeitraum von mindestens zwei oder mindestens drei Tagen hergerichtet. Längere Anwendungszeiträume sind ebenso

möglich.

Im Folgenden werden bevorzugte Ausführungsformen des erfindungsgemäßen TTS anhand von experimentellen Beispielen beschrieben und deren Eigenschaften hinsichtlich der Stabilität bestimmt.

**Erläuterungen der Figuren 1-7:**

Im Folgenden bezeichnet der Parameter "n" die Anzahl der Wiederholungen der durchgeführten Messungen, welche zu gemittelten Ergebnissen führen.

Figur 1: Schematischer Querschnitt eines Rivastigmin TTS mit Membran (nicht maßstabsgerecht).

Figur 2: Diagramm bezüglich des maximalen kalten Fluss (Cold Flow) der Wirkstoffschichten nach 9-wöchiger Lagerung bei 25 °C/60% r.F. und 40 °C/75% r.F. (n = 2).

Figur 3: Diagramm bezüglich der Klebkraft der gemäß den Beispielen hergestellten Klebeschichten (n = 3).

Figur 4: In vitro-Permeationsprofil von Rivastigmin aus Exelon® (n = 6).

Figur 5: In vitro-Permeationsprofile von Rivastigmin aus Zweischichtlaminaten mit und ohne Membran (n = 4).

Figur 6: *In* vitro-Permeationsprofile von Rivastigmin aus Zweischichtlaminaten mit Klebeschichten basierend auf einem Polyacrylat-Kleber (n = 4).

Figur 7: In vitro-Permeationsprofile von Rivastigmin aus Zweischichtlaminaten mit verschiedenen Membranen und Klebeschichten im Vergleich zum Exelon® (n ≥ 4).

Figur 8: In vitro-Permeationsprofile von Rivastigmin aus Zweischichtlaminaten mit verschiedenen Membranen (n ≥ 4).

Figur 9: In vitro-Freisetzungsprofil von Rivastigmin aus Zweischichtlaminaten mit verschiedenen Membranen (n ≥ 4).

**Beispiele**

Die in den nachfolgenden Formulierungsbeispielen eingesetzten Komponenten können folgendermaßen näher beschrieben werden:

Tabelle 1: Übersicht der Komponenten der Formulierungsbeispiele

| Komponentenbezeichnung | Chemische Beschreibung | Funktion |
|---|---|---|
| Duro-Tak® 87-4098 | Acrylat/Vinylacetat Copolymer | Matrixpolymer |
| Duro-Tak® 87-9088 | Acrylat Copolymer | Matrixpolymer |
| Cab-O-Sil® | Pyrogenes Siliziumdioxid | Gelbildner |
| Oppanol® B10 | Polyisobutylen ($M_V$= ca. 4x10$^4$ g/mol) | Haftkleber |
| Oppanol® B50 SF | Polyisobutylen ($M_V$= ca. 4x10$^5$ g/mol) | Haftkleber |
| Indopol H-100, H-1900, H-6.000, H-18.000 | Polybuten ($M_N$ = 910, 2.500, 4.200 bzw. 6.000 g/mol) | Klebrigmacher |
| Eudragit® E100 | Acrylat Copolymer | Matrixpolymer |

**Beispiel 1**

Herstellung verschiedener Wirkstoffschicht-Formulierungen

Es wurden drei unterschiedliche Wirkstoffschicht-Formulierungen, welche Rivastigmin Base enthalten, hergestellt. Eine Übersicht der Bestandteile der verschiedenen Formulierungen ist in Tabelle 2 wiedergegeben.

Tabelle 2: Verwendete Wirkstoffschichten

| Charge | Wirkstoffschicht (R) | Matrixgewicht mg/10cm$^2$ |
|---|---|---|
| 010RIDTDS | 40 % Rivastigmin; 60 % DT® 87-9088 | 64 |
| 011 RIDTDS | 40 % Rivastigmin; 60 % DT® 87-4098 | 62 |
| 012RIDTDS | 40 % Rivastigmin; 60 % Eudragit® E100 | 54 |

In Figur 2 sind die Ergebnisse zur physikalischen Stabilität (Cold flow) der obenstehenden Rerservoir-Formulierungen wiedergegeben. Die Formulierung mit DT® 87-9088 (010RIDTDS) zeigt nach 9-wöchiger Lagerung den geringsten Cold flow und eignet sich daher besonders als Matrixbildner für die Wirkstoffschicht.

**Beispiel 2**

Es wurden sechs unterschiedliche Formulierungen der Klebeschicht hergestellt. Eine Übersicht der Bestandteile der verschiedene Formulierungen ist in Tabelle 3 wiedergeben.

Tabelle 3: Verwendete Klebeschichten

| Charge | Klebeschicht (A) | Matrixschicht mg/10 cm$^2$ |
|---|---|---|
| 019RIDTDS | 17,91 % Oppanol® B10 SFN; 31,84 % Oppanol® B50SF; 29,85 % Indopol® H-100; 19,90 % Indopol® H-1900; 0,50 % Cab-O-Sil® M5P | 30 |
| 005RIDTDS | 24,88 % Oppanol® B10 SFN; 24,88 % Oppanol® B50SF; 24,88 % Indopol® H-100; 24,88 % Indopol® H-1900; 0,50 % Cab-O-Sil® M5P | 32 |
| 013RIDTDS | 24,88 % Oppanol® B10 SFN; 24,88 % Oppanol® B50SF; 24,88 % Indopol® H-100; 24,88 % Indopol® H-6000; 0,50 % Cab-O-Sil® M5P | 32 |
| 014RIDTDS | 24,88 % Oppanol® B10 SFN; 24,88 % Oppanol® B50SF; 24,88 % Indopol® H-100; 24,88 % Indopol® H-18000; 0,50 % Cab-O-Sil® M5P | 30 |
| 015RIDTDS | 29,85 % Oppanol® B10 SFN; 19,90 % Oppanol® B50SF; 19,90 % Indopol® H-1900; 29,85 % Indopol® H-18000; 0,50 % Cab-O-Sil® M5P | 35 |
| C006RIVTDS | 64,5 % Oppanol® B10 SFN/B50SF (4/6); 35,0 % Paraffin; 0,5 % Cabo-O-Sil® | 30 |

In Figur 3 sind die Ergebnisse von Messungen der Klebkraft der hergestellten Klebeschichten abgebildet. Daraus geht hervor, dass durch Zusatz von Indopol® zum Oppanol®-Kleber die Klebkraft erheblich verbessert wird, verglichen mit der Verwendung von Paraffin. Darüber hinaus kann die Klebkraft durch die gezielte Auswahl der Molekulargewichte der eingesetzten Polymere weiter erhöht und gesteuert werden.

**Beispiel 3**

Schließlich wurden sechs unterschiedliche Chargen von TTS-Formulierungen hergestellt. Eine Übersicht über die Zusammensetzung der verschiedenen Chargen ist in Tabelle 4 zusammengestellt.

Tabelle 4: Verwendete Chargen:

| **Charge** | **Wirkstoffschicht (R)** | **Klebeschicht (A)** | **Membran** | **Matrixgewicht mg/ 10 cm$^2$** |
|---|---|---|---|---|
| 015/017 RI DTDS ohne Membran (Referenz) | 40% Rivastigmin; 60% DT® 87-9088 | 29,85% Oppanol® B10 SFN<br>19,90% Oppanol® B50SF<br>19,90% Indopol® H-1900<br>29,85% Indopol® H-18000<br>0,50% Cap-O-Sil M5P | ---- | 015RIDTDS (A): 35<br>017RIDTDS (R): 60 |
| 015/017 RIDTDS CoTran 9719 | 40% Rivastigmin; 60% DT® 87-9088 | 29,85% Oppanol® B10 SFN<br>19,90% Oppanol® B50SF<br>19,90% Indopol® H-1900<br>28,85% Indopol® H-18000<br>0,50% Cap-O-Sil M5P | CoTran™ 9719 | 015RIDTDS (A): 35<br>017RIDTDS (R): 60 |
| 009/010 RIDTDS Celgard 2400 | 40% Rivastigmin;<br>60% DT® 87-9088 | 10% Rivastigmin;<br>90% DT® 87-2516 | Celgard® 2400 | 009RIDTDS (A): 33<br>010RIDTDS (R): 64 |
| 009/010 RIDTDS Solupor 10P05A | 40% Rivastigmin;<br>60% DT® 87-9088 | 10% Rivastigmin;<br>90% DT® 87-2516 | Solupor® 10P05A | 009RIDTDS (A): 33<br>010RIDTDS (R):64 |
| 029/030 RIDTDS Solupor 10P05A | 40% Rivastigmin; 60% DT® 87-9088 | 20% Oppanol® B10 SFN<br>30% Oppanol® B50SF<br>30% Indopol® H-100<br>20% Indopol® H-1900 | Solupor® 10P05A | 029RIDTDS (A):34<br>030RIDTDS (R):61 |
| 028/030 RIDTDS CoTran 9719 | 40% Rivastigmin; 60% DT® 87-9088 | 30% Oppanol® B10 SFN<br>20% Oppanol® B50SF<br>20% Indopol® H-1900<br>30% Indopol® H-18000 | CoTran™ 9719 | 028RIDTDS (A):28<br>030RIDTDS<br>(R):61 |
| 070/071 RIDTDS CoTran 9720 | 40% Rivastigmin; 60% DT® 87-9088 | 30% Oppanol® B10 SFN<br>20% Oppanol® B50SF<br>20% Indopol® H-1900<br>30% Indopol® H-18000 | CoTran™ 9720 | 070RIDTDS (A): 30<br>071RIDTDS (R): 60 |

Herstellungsverfahren

1. Herstellung der Wirkstoffschicht

Der Acrylat Kleber wurde vorgelegt und Rivastigmin und Ethylacetat dazu gewogen. Anschliessend wurden die Komponenten in ausreichend Ethylacetat mittels eines Rührers gemischt, sodass eine streichfähige, homogene Beschichtungsmasse entsteht.

Die homogene Beschichtungsmasse wurde auf einer silikonisierten Folie ("intermediate liner") als dünner Film aufgetragen. Der Matrixfilm wurde bei 60°C/20 min und 80°C/5 min getrocknet und anschliessend mit einer Deckschicht aus PET kaschiert.

Anschließend wurde der "intermediate liner" abgezogen und die Kontrollmembran zukaschiert.

2. Herstellung der Adhäsivschicht und des Gesamtlaminats

Die Polyisobutylenkleber wurden zusammen eingewogen und gemischt. Anschliessend wurden unter Rühren Heptan und Cab-O-Sil® zugegeben und so lange gerührt bis die Masse homogen war.

Die Masse wurde auf eine Abziehschicht ("release liner") als dünner Film aufgetragen und anschliessend werden die Lösemittel bei 60°C/20 min und 80°C/5 min abgezogen. Nach der Trocknung wird das Laminat mit der Wirkstoffschichtkaschiert.

Aus dem erhaltenen Laminat wurden Pflaster geeigneter Grösse ausgestanzt.

3. In vitro-Maus-Hautpermeationstest

Mit den Chargen aus Tabelle 4 und dem kommerziellen Produkt Exelon® TDS, einer Eintagesformulierung, wurden in vitro-Maus-Hautpermeationstests durchgeführt.

Die Ergebnisse dieser Tests sind in den Figuren 4 bis 7 wiedergegeben. Figur 4 zeigt das Permeationsprofil von Rivastigmin aus dem kommerziell verfügbaren Exelon® TDS. Durch den Kurvenverlauf in Figur 4 wird deutlich, dass die Abgaberate von Rivastigmin bereits nach 24 Stunden deutlich abnimmt. Dieses System gewährleistet daher keine kontinuierliche und gleichmäßige Wirkstoffabgabe über einen Anwendungszeitraum von mehr als 24 Stunden.

Aus Figur 5 geht hervor, dass nur durch die zwischenliegende Membran eine mehrtägige kontinuierliche und gleichmäßige Wirkstoffabgabe ermöglicht wird.

Die Formulierungsansätze 009/010RIDTDS Celgard® 2400 und 009/010RIDTDS Solupor® 10P05A, die Polyacrylat als Matrixbildner für die Klebeschicht enthalten, wiesen überraschenderweise eine im Vergleich zum Exelon® TDS ähnliche Kinetik auf (vgl. Figur 4 und 6). Daraus geht hervor, dass sich Polymere aus der Gruppe der Polyacrylate und dessen Copolymere nicht als Matrixbildner für die Klebeschicht eignen, da sie, selbst bei zwischenliegender Membran, keine ausreichende Steuerung der Wirkstoffabgabe über die mehrtägige Applikationszeit gewährleisten.

Aus den Figuren 5 und 7 geht hervor, dass durch die Kombination einer die Freisetzung des Wirkstoffs kontrollierenden Membran und einer Klebeschicht basierend auf einem Polyisobutylen/Polybuten-Gemisch eine Steuerung der Arzneistoffabgabe über ein mehrtägiges Zeitintervall ermöglichen.

Figur 8 zeigt, dass mit den beiden Formulierungen "C008RIDTDS" und "070/071 RIDTDS CoTran 9720" ein gleichmäßig lineares Permeationsprofil über sieben Tage erreicht wird. Diese Formulierungen sind daher auch als 5-Tagespflaster, als 6-Tagespflaster und als 7-Tagespflaster besonders geeignet. Die Formulierung "C008RIDTDS" stammt aus einer Klinikcharge und hat die gleiche Zusammensetzung wie die in Beispiel 4 beschriebene Formulierung I (060/062RIDTDS_Cotran9719).

4. In vitro-Freisetzung

Die in vitro-Freisetzung wurde mit der "Disk assembly Method" nach Ph. Eur. 5.0 <2.9.4> bestimmt.

Figur 9 zeigt, dass die beiden Formulierungen "C008RIDTDS" und "070/071 RIDTDS CoTran 9720" eine gleichmäßige Freisetzung des Wirkstoffs über wenigstens sieben Tage zeigen.

**Beispiel 4: Stabilitätstest**

Nach dem oben angegebenen Verfahren wurden noch zwei weitere Formulierungen hergestellt:

Tabelle 5

| | Formulierung I (060/062R)DTDS_ Cotran9719) | Formulierung II (058/062R)DTDS_ Cotran9728) |
|---|---|---|
| **Wirkstoffschicht 60 g/m$^2$** | Rivastigmin (40%)<br>DT® 87-9088 (60%) | Rivastigmin (40%)<br>DT® 87-9088 (60%) |
| **Membran** | CoTran™ 9719 | CoTran™ 9728 |
| **Adhäsivschicht 30 g/m$^2$** | Oppanol® B10 SFN (30%)<br>Oppanol® B50 SF (20%)<br>Indopol® H-1900 (20%)<br>Indopol® H-18000 (30%) | Oppanol® B10 SFN (30%)<br>Oppanol® B50 SF (40%)<br>Indopol® H-1900 (30%) |

Die ausgestanzten TTS wurden in Beutel aus Aluminium-Verbundfolien versiegelt und jeweils mindestens einen Monat lang bei 25°C und 60 % relativer Luftfeuchtigkeit, beziehungsweise bei 40°C und 75 % relativer Luftfeuchtigkeit gelagert. Anschließend wurde der Gehalt eventuell entstandener Verunreinigungen als Folge der Zersetzung von Rivastigmin mittels HPLC und UV-Absorption ermittelt.

Die erfindungsgemäßen TTS zeigten eine ausgezeichnete Stabilität über mehrere Monate. Nach Lagerung wurden nur sehr geringe Mengen an Verunreinigungen/ Zersetzungsprodukten nachgewiesen, obwohl die Wirkstoffschicht keine Antioxidantien enthielt. Dies wurde durch den Einsatz einer Polymermatrix ohne Hydroxylgruppen und ohne Carboxylgruppen erreicht.

Die Ergebnisse sing in der folgenden Tabelle 6 zuzammengefasst:

Tabelle 6: Verunreinigungen nach Lagerung

| | | Temperatur | Initial | 1 Monat | 2 Monate | 3 Monate |
|---|---|---|---|---|---|---|
| | **Charge** | **°C / % r.F.** | | | | z" |
| Reinheit n=3 | 060/062 RI DTDS _CoTran9719 | 25 / 60 | **Imp. 1:** 0,04% **(< RL); Imp. 4:** 0,02% (≤ **RL); Imp. 5:** 0,05% **(< RL);** → Summe: 0,0% | **Imp. 1:** 0,05% **(< RL); Imp. 4:** 0,06% **(< RL); Imp. 5:** 0,14%; → Summe: 0,14% | **Imp. 1:** 0,02% **(< RL); Imp. 4:** 0,07% **(< RL); Imp. 5: 0,18%** → Summe: 0,18% | **Imp. 1:** 0,02% **(< RL); RRT=0,84:** 0,01% **(<RL); Imp. 2:** 0,02% **(< RL); Imp. 4:** 0,06% **(<RL); Imp. 5: 0,17%** → Summe: 0,17% |
| | | 40 / 75 | | Imp. **1:** 0,04% **(< RL); RRT=0,85:** 0,06% (≤ RL); **Imp. 2:** 0,02% **(< RL); Imp. 4: 0,11%;** Imp. 5: **0,23%** → Summe: 0,34% | | |
| | 058/062RIDTDS _CoTran9728 | 25 / 60 | **Imp. 1:** 0,04% **(< RL); Imp. 4:** 0,02% **(< RL); Imp. 5:** 0,05% **(< RL);** → Summe: 0,0% | **Imp. 1:** 0,05% **(< RL); Imp. 4:** 0,05% **(< RL); Imp. 5:** 0,15% → Summe: 0,15% | **Imp. 1:** 0,02% **(< RL); Imp. 4:** 0,06% **(< RL); Imp. 5: 0,18%** → Summe: **0,18%** | **Imp. 1:** 0,02% **(< RL); RRT=0,84:** 0,02% **(<RL); Imp. 2:** 0,02% **(< RL); Imp. 4:** 0,09% **(<RL); Imp. 5: 0,18%;** → Summe: **0,18%** |
| | | 40 / 75 | | **Imp. 1:** 0,05% **(< RL); RRT=0,85:** 0,04% (< RL); **Imp. 2:** 0,03% **(< RL); Imp. 4:** 0,09% **(< RL); Imp. 5: 0,22%** → Summe: 0,22% | | |
| RL="Reporting Limit" (Nachweisgrenze)<br>RRT=relative Retentionszeit (HPLC) | | | | | | |

## Patentansprüche

**1.** Transdermales therapeutisches System zur Verabreichung eines Wirkstoffs durch die Haut umfassend die zueinander in der folgenden Reihenfolge angeordneten Schichten:

a) eine Deckschicht;
b) eine Wirkstoffschicht umfassend eine den Wirkstoff enthaltende Polymermatrix;
c) eine die Freisetzung des Wirkstoffs kontrollierende Membran;
d) eine Klebeschicht umfassend einen Haftkleber; und
e) eine Abziehschicht;

wobei der Wirkstoff Rivastigmin oder ein physiologisch verträgliches Salz, Hydrat, oder Solvat davon ist, **dadurch gekennzeichnet**, (i) dass das Polymer/die Polymere der Polymermatrix keine freien Hydroxylgruppen und keine

freien Carboxylgruppen enthält/enthalten, und (ii) dass die Wirkstoffschicht keine freien Hydroxylgruppen und keine freien Carboxylgruppen enthält.

2. Transdermales therapeutisches System nach Anspruch 1, **dadurch gekennzeichnet**, (i) dass es keine Tocopherole enthält, (ii) dass es kein Antioxidationsmittel enthält, das ausgewählt ist aus der Gruppe bestehend aus Tocopherolen, Butylhydroxdyanisol und Butylhydroxytoluol, und/oder (iii) dass es kein Antioxidationsmittel enthält.

3. Transdermales therapeutisches System nach Anspruch 1 oder 2, **dadurch gekennzeichnet, dass** der Haftkleber ein Polyisobutylen oder ein Gemisch von mehreren Polyisobutylenen enthält, und die Klebeschicht gegebenenfalls weiterhin ein Polybuten oder ein Gemisch von mehreren Polybutenen enthält.

4. Transdermales therapeutisches System nach einem der Ansprüche 1 bis 3, **dadurch gekennzeichnet, dass** der Haftkleber aus mindestens zwei Polyisobutylenen mit unterschiedlichen mittleren Molekulargewichten und mindestens zwei Polybutenen mit unterschiedlichen mittleren Molekulargewichten besteht, wobei das erste Polyisobutylenpolymer vorzugsweise ein mittleres Molekulargewicht $M_v$ von 40.000 g/mol hat, und das zweite Polyisobutylenpolymer vorzugsweise ein mittleres Molekulargewicht $M_v$ von 400.000 g/mol hat; und/oder wobei das erste Polybutenpolymer vorzugsweise ein mittleres Molekulargewicht $M_n$ im Bereich von 700 - 2.800 g/mol hat, und das zweite Polybutenpolymer vorzugsweise ein mittleres Molekulargewicht $M_n$ im Bereich von 2.200 - 6.500 g/mol hat.

5. Transdermales therapeutisches System nach einem der Ansprüche 1 bis 4, wobei die Wirkstoffschicht 30 - 50 Gew.-% Wirkstoff und 50 - 70 Gew.-% Polymermatrix, bezogen auf das Gesamtgewicht der Wirkstoffschicht, enthält.

6. Transdermales therapeutisches System nach einem der Ansprüche 1 bis 5, wobei die Polymermatrix der Wirkstoffschicht wenigstens ein Polymer und/oder Copolymer ohne freie Hydroxylgruppen und ohne freie Carboxylgruppen umfasst, ausgewählt aus der Gruppe bestehend aus Polyacrylaten, Acrylat-Vinylacetat Copolymeren, Polyisobutylen, Styrol-Butadien Copolymeren und Mischungen davon.

7. Transdermales therapeutisches System nach Anspruch 1, umfassend:

a) eine Deckschicht,
b) ein auf der Deckschicht befindliche Wirkstoffschicht enthaltend 30 - 50 Gew.-% Wirkstoff und 50 - 70 % Polymermatrix, bezogen auf das Gesamtgewicht der Wirkstoffschicht, wobei die Polymermatrix im wesentlichen aus einem Acrylat-Polymer ohne freie Hydroxylgruppen und ohne freie Carboxylgruppen, oder im wesentlichen aus einem Acrylat-Copolymer ohne freie Hydroxylgruppen und ohne freie Carboxylgruppen besteht, und wobei der Wirkstoff Rivastigmin oder ein physiologisch verträgliches Salz, Hydrat oder Solvat davon ist;
c) eine auf der Wirkstoffschicht befindliche, die Freisetzung des Rivastigmins kontrollierende Membran;
d) eine auf der Membran befindliche Klebeschicht bestehend aus 0 - 1 Gew.-% Siliziumdioxid und 99 - 100 Gew.-% eines Gemisches aus einem Polyisobutylenpolymer mit einem mittleren Molekulargewicht Mv von 40.000 g/mol, einem Polyisobutylenpolymer mit einem mittleren Molekulargewicht Mv von 400.000 g/mol, einem Polybutenpolymer mit einem mittleren Molekulargewicht $M_N$ von 2.500 g/mol und einem Polybutenpolymer mit einem mittleren Molekulargewicht $M_N$ von 6.000 g/mol; und
e) eine auf der Klebeschicht befindliche Abziehschicht.

8. Transdermales therapeutisches System nach Anspruch einem der Ansprüche 1 bis 7, **dadurch gekennzeichnet, dass** es zur Anwendung über wenigstens 7 Tage geeignet ist.

9. Transdermales therapeutisches System nach einem der Ansprüche 1 bis 8, **dadurch gekennzeichnet, dass** es über einen Zeitraum von wenigstens 72 Stunden eine im wesentlichen lineare Hautpermeation des Wirkstoffs zeigt, gemessen durch einen in-vitro-Hautpermeationstest.

10. Verfahren zur Herstellung eines transdermalen Systems nach einem der Ansprüche 1 bis 9, umfassend

i) die Herstellung einer die Wirkstoffschicht enthaltenden Komponente, enthaltend die Deckschicht und die Wirkstoffschicht, welches sich auf der Seite der Deckschicht befindet, welche als die der Haut zugewandten Seite bestimmt ist;
ii) die Herstellung einer die Klebeschicht enthaltenden Komponente, enthaltend die Abziehschicht und die sich auf der Abziehschicht befindliche Klebeschicht und gegebenenfalls eine die Freisetzung des Wirkstoffs kontrollierenden Membran;

iii) das aufeinander Laminieren der Komponenten aus i) und ii), so dass Deck- und Abziehschicht im Querschnitt des fertigen TTS die gegenüberliegenden, äußersten Schichten darstellen.

**11.** Transdermales therapeutisches System nach einem der Ansprüche 1 bis 9 zur Verwendung in der Behandlung von Alzheimer und Parkinsondemenz, wobei das transdermale therapeutische System vorzugsweise über einen Zeitraum von wenigstens 72 Stunden verabreicht wird.

**12.** Verwendung eines Polymers oder Copolymers, das keine freien Hydroxylgruppen und keine freien Carboxylgruppen aufweist, zur Stabilisierung von Rivastigmin in einem TTS, oder zur Verringerung des Abbaus von Rivastigmin in einem TTS.

**13.** Verfahren zur Stabilisierung von Rivastigmin in einem TTS oder zur Verringerung des Abbaus von Rivastigmin in einem TTS, **dadurch gekennzeichnet, dass** man Rivastigmin, oder ein Salz oder Solvat davon, in eine Polymermatrix einbettet, die keine freien Hydroxylgruppen und keine freien Carboxylgruppen aufweist, und dass man Rivastigmin, oder ein Salz oder Solvat davon, in eine Wirkstoffschicht einbringt, die keine freien Hydroxylgruppen und keine freien Carboxylgruppen enthält.

**14.** Transdermales therapeutisches System nach einem der Ansprüche 1 bis 9, oder transdermales therapeutisches System zur Verwendung nach Anspruch 11, **dadurch gekennzeichnet, dass** es eine durchschnittliche Freisetzungsrate in vivo zwischen 0,1 $mg/d/cm^2$ und 2 $mg/d/cm^2$, und/oder von wenigstens 0,3 $mg/d/cm^2$ aufweist.

## Claims

**1.** A transdermal therapeutic system for administering an active ingredient through the skin comprising the layers arranged to each other in the following order:

a) a cover layer;
b) an active ingredient layer comprising a polymer matrix containing the active ingredient;
c) a membrane controlling the release of the active ingredient;
d) an adhesive layer comprising pressure-sensitive adhesive; and
e) a stripping layer;

wherein the active ingredient is rivastigmine or a physiologically acceptable salt, hydrate, or solvate thereof, **characterized in that** (i) the polymer(s) of the polymer matrix contain(s) no free hydroxyl groups and no free carboxyl groups, and (ii) the active ingredient layer contains no free hydroxyl groups and no free carboxyl groups.

**2.** The transdermal therapeutic system according to claim 1, **characterized in that** (i) it contains no tocopherols, (ii) it contains no antioxidant selected from the group consisting of tocopherols, butylhydroxyanisole, and butylhydroxytoluene, and/or (iii) it contains no antioxidant.

**3.** The transdermal therapeutic system according to claim 1 or 2, **characterized in that** the pressure-sensitive adhesive contains a polyisobutylene or a mixture of several polyisobutylenes, and the adhesive layer optionally further contains a polybutene or a mixture of several polybutenes.

**4.** The transdermal therapeutic system according to any one of claims 1 to 3, **characterized in that** the pressure-sensitive adhesive consists of at least two polyisobutylenes of different average molecular weights and at least two polybutenes of different average molecular weights, wherein the first polyisobutylene polymer preferably has an average molecular weight $M_v$ of 40,000 g/mol, and the second polyisobutylene polymer preferably has an average molecular weight $M_v$ of 400,000 g/mol; and/or wherein the first polybutene polymer preferably has an average molecular weight $M_n$ in the range of 700-2,800 g/mol, and the second polybutene polymer preferably has an average molecular weight $M_n$ in the range of 2,200-6,500 g/mol.

**5.** The transdermal therapeutic system according to any one of claims 1 to 4, wherein the active ingredient layer contains 30-50% by weight of the active ingredient and 50-70% by weight of the polymer matrix based on the total weight of the active ingredient layer.

**6.** The transdermal therapeutic system according to any one of claims 1 to 5, wherein the polymer matrix of the active

ingredient layer comprises at least one polymer and/or copolymer without any free hydroxyl groups and without any free carboxyl groups selected from the group consisting of polyacrylates, acrylate-vinyl acetate copolymers, polyisobutylene, styrene-butadiene copolymers and mixtures thereof.

**7.** The transdermal therapeutic system according to claim 1 comprising:

a) a cover layer,
b) an active ingredient layer on the cover layer that contains 30-50% by weight of active ingredient and 50-70% of polymer matrix based on the total weight of the active ingredient layer, wherein the polymer matrix substantially consists of an acrylate polymer without any free hydroxyl groups and without any free carboxyl groups or substantially of an acrylate copolymer without any free hydroxyl groups and without any free carboxyl groups, and wherein the active ingredient is rivastigmine or a physiologically acceptable salt, hydrate, or solvate thereof;
c) a membrane on the active ingredient layer that controls the release of the rivastigmine;
d) an adhesive layer on the membrane that consists of 0-1 % by weight of silica and 99-100% by weight of a mixture of a polyisobutylene polymer having an average molecular weight $M_v$ of 40,000 g/mol, a polyisobutylene polymer having an average molecular weight $M_v$ of 400,000 g/mol, a polybutene polymer having an average molecular weight $M_n$ of 2,500 g/mol, and a polybutene polymer having an average molecular weight $M_n$ of 6,000 g/mol; and
e) a stripping layer on the adhesive layer.

**8.** The transdermal therapeutic system according to any one of claims 1 to 7, **characterized in that** it is suitable for use over at least 7 days.

**9.** The transdermal therapeutic system according to any one of claims 1 to 8, **characterized in that** it exhibits a substantially linear skin permeation of the active ingredient over a period of at least 72 hours, measured by an in vitro skin permeation test.

**10.** A method for preparing a transdermal system according to any one of claims 1 to 9, comprising

i) preparing a component containing the active ingredient layer that contains the cover layer and the active ingredient layer that is located on the side of the cover layer intended as the side facing the skin;
ii) preparing a component containing the adhesive layer that contains the stripping layer and the adhesive layer located on the stripping layer and optionally a membrane controlling the release of the active ingredient;
iii) laminating onto each other the components of i) and ii), so that the cover and stripping layer represent the opposite outer layers in the cross section of the finished TTS.

**11.** The transdermal therapeutic system according to any one of claims 1 to 9 for use in the treatment of Alzheimer and Parkinson dementia, wherein the transdermal therapeutic system is preferably administered over a period of at least 72 hours.

**12.** Use of a polymer or copolymer containing no free hydroxyl groups and no free carboxyl groups to stabilize rivastigmine in a TTS, or to reduce decomposition of rivastigmine in a TTS.

**13.** A method for stabilizing rivastigmine in a TTS or for reducing decomposition of rivastigmine in a TTS, **characterized in that** rivastigmine, or a salt or solvate thereof is embedded into a polymer matrix containing no free hydroxyl groups and no free carboxyl groups and that rivastigmine, or a salt or solvate thereof is inserted into an active ingredient layer containing no free hydroxyl groups and no free carboxyl groups.

**14.** The transdermal therapeutic system according to any one of claims 1 to 9, or the transdermal therapeutic system for use according to claim 11, **characterized in that** it has an average in vivo release rate between 0.1 $mg/d/cm^2$ and 2 $mg/d/cm^2$, and/or of at least 0.3 $mg/d/cm^2$.

**Revendications**

**1.** Système thérapeutique transdermique destiné à l’administration d’un principe actif à travers la peau comprenant les couches disposées les unes par rapport aux autres dans l’ordre suivant :

a) une couche de recouvrement ;
b) une couche de principe actif comprenant une matrice polymère contenant le principe actif ;
c) une membrane régulant la libération du principe actif ;
d) une couche adhésive comprenant un adhésif sensible à la pression ; et
e) une couche pelable ;
le principe actif étant la rivastigmine ou un sel, hydrate ou solvate physiologiquement tolérable de celle-ci, caractérisé (i) en ce que le polymère/les polymères de la matrice polymère ne contient/contiennent pas de groupes hydroxyle libres et pas de groupes carboxyle libres, et (ii) en ce que la couche de principe actif ne contient pas de groupes hydroxyle libres et pas de groupes carboxyle libres.

**2.** Système thérapeutique transdermique selon la revendication 1, caractérisé (i) en ce qu'il ne contient pas de tocophérols, (ii) en ce qu'il ne contient pas d'antioxydant, qui est choisi dans le groupe se composant de tocophérols, butylhydroxyanisol et butylhydroxytoluène, et/ou (iii) en ce qu'il ne contient pas d'antioxydant.

**3.** Système thérapeutique transdermique selon la revendication 1 ou 2, **caractérisé en ce que** l'adhésif sensible à la pression contient un polyisobutylène ou un mélange de plusieurs polyisobutylènes, et la couche adhésive contient le cas échéant en outre un polybutène ou un mélange de plusieurs polybutènes.

**4.** Système thérapeutique transdermique selon l'une quelconque des revendications 1 à 3, **caractérisé en ce que** l'adhésif sensible à la pression se compose d'au moins deux polyisobutylènes présentant des masses moléculaires moyennes différentes et d'au moins deux polybutènes présentant des masses moléculaires moyennes différentes, le premier polymère polyisobutylène présentant de préférence une masse moléculaire moyenne $M_v$ de 40 000 g/mol, et le deuxième polymère polyisobutylène présentant de préférence une masse moléculaire moyenne $M_v$ de 400 000 g/mol ; et/ou le premier polymère polybutène présentant de préférence une masse moléculaire moyenne $M_n$ se situant dans la plage allant de 700 à 2800 g/mol, et le deuxième polymère polybutène présentant de préférence une masse moléculaire moyenne $M_n$ se situant dans la plage allant de 2200 à 6500 g/mol.

**5.** Système thérapeutique transdermique selon l'une quelconque des revendications 1 à 4, la couche de principe actif contenant de 30 à 50 % en poids de principe actif et de 50 à 70 % en poids de matrice polymère, par rapport au poids total de la couche de principe actif.

**6.** Système thérapeutique transdermique selon l'une quelconque des revendications 1 à 5, la matrice polymère de la couche de principe actif comprenant au moins un polymère et/ou un copolymère sans groupe hydroxyle libre et sans groupe carboxyle libre, choisie dans le groupe se composant de polyacrylates, copolymères acrylate-acétate de vinyle, polyisobutylène, copolymères styrène-butadiène et des mélanges de ceux-ci.

**7.** Système thérapeutique transdermique selon la revendication 1, comprenant :

a) une couche de recouvrement,
b) une couche de principe actif se trouvant sur la couche de recouvrement contenant de 30 à 50 % en poids de principe actif et de 50 à 70 % de matrice polymère, par rapport au poids total de la couche de principe actif, la matrice polymère se composant sensiblement d'un polymère acrylate sans groupe hydroxyle libre et sans groupe carboxyle libre, ou sensiblement d'un copolymère acrylate sans groupe hydroxyle libre et sans groupe carboxyle libre, et le principe actif étant la rivastigmine ou un sel, hydrate ou solvate physiologiquement tolérable de celle-ci ;
c) une membrane régulant la libération de la rivastigmine se trouvant sur la couche de principe actif ;
d) une couche adhésive se trouvant sur la membrane se composant de 0 à 1 % en poids de dioxyde de silicium et de 99 à 100 % en poids d'un mélange se composant d'un polymère polyisobutylène présentant une masse moléculaire moyenne $M_V$ de 40 000 g/mol, un polymère polyisobutylène présentant une masse moléculaire moyenne $M_V$ de 400 000 g/mol, un polymère polybutène présentant une masse moléculaire moyenne $M_N$ de 2500 g/mol et un polymère polybutène présentant une masse moléculaire moyenne $M_N$ de 6000 g/mol ; et
e) une couche pelable se trouvant sur la couche adhésive.

**8.** Système thérapeutique transdermique selon la revendication l'une quelconque des revendications 1 à 7, **caractérisé en ce qu'**il convient à une utilisation d'au moins 7 jours.

**9.** Système thérapeutique transdermique selon l'une quelconque des revendications 1 à 8, **caractérisé en ce qu'**il présente sur une période d'au moins 72 heures une pénétration à travers la peau sensiblement linéaire du principe

actif, mesurée par un test de pénétration cutanée *in vitro.*

**10.** Procédé de préparation d'un système transdermique selon l'une quelconque des revendications 1 à 9, comprenant

i) la préparation d'un composant contenant la couche de principe actif, contenant la couche de recouvrement et la couche de principe actif, lequel se trouve du côté de la couche de recouvrement, laquelle est déterminée comme le côté orienté vers la peau ;
ii) la préparation d'un composant contenant la couche adhésive, contenant la couche pelable et la couche adhésive se trouvant sur la couche pelable et le cas échéant une membrane régulant la libération du principe actif ;
iii) la stratification des composants les uns sur les autres à partir de i) et ii), de sorte que la couche de recouvrement et la couche pelable en section transversale du STT final représentent les couches opposées les plus externes.

**11.** Système thérapeutique transdermique selon l'une quelconque des revendications 1 à 9 destiné à l'utilisation dans le traitement de la maladie d'Alzheimer et de la démence associée à la maladie de Parkinson, le système thérapeutique transdermique étant administré de préférence sur une durée d'au moins de 72 heures.

**12.** Utilisation d'un polymère ou d'un copolymère, qui ne présente pas de groupes hydroxyle libres et pas de groupes carboxyle libres, destinée à la stabilisation de la rivastigmine dans un STT, ou destinée à la diminution de l'élimination de la rivastigmine dans un STT.

**13.** Procédé de stabilisation de la rivastigmine dans un STT ou de diminution de l'élimination de la rivastigmine dans un STT, **caractérisé en ce qu'**on incorpore de la rivastigmine ou un sel ou solvate de celle-ci, dans une matrice polymère, qui ne présente pas de groupes hydroxyle libres et pas de groupes carboxyle libres, et **en ce qu'**on introduit de la rivastigmine ou un sel ou solvate de celle-ci dans une couche de principe actif, qui ne contient pas de groupes d'hydroxyle libres et pas de groupes de carboxyle libres.

**14.** Système thérapeutique transdermique selon l'une quelconque des revendications 1 à 9, ou système thérapeutique transdermique destiné à l'utilisation selon la revendication 11, **caractérisé en ce qu'**il présente une vitesse de libération moyenne *in vivo* comprise entre 0,1 $mg/d/cm^2$ et 2 $mg/d/cm^2$, et/ou d'au moins 0,3 $mg/d/cm^2$.

Figur 1

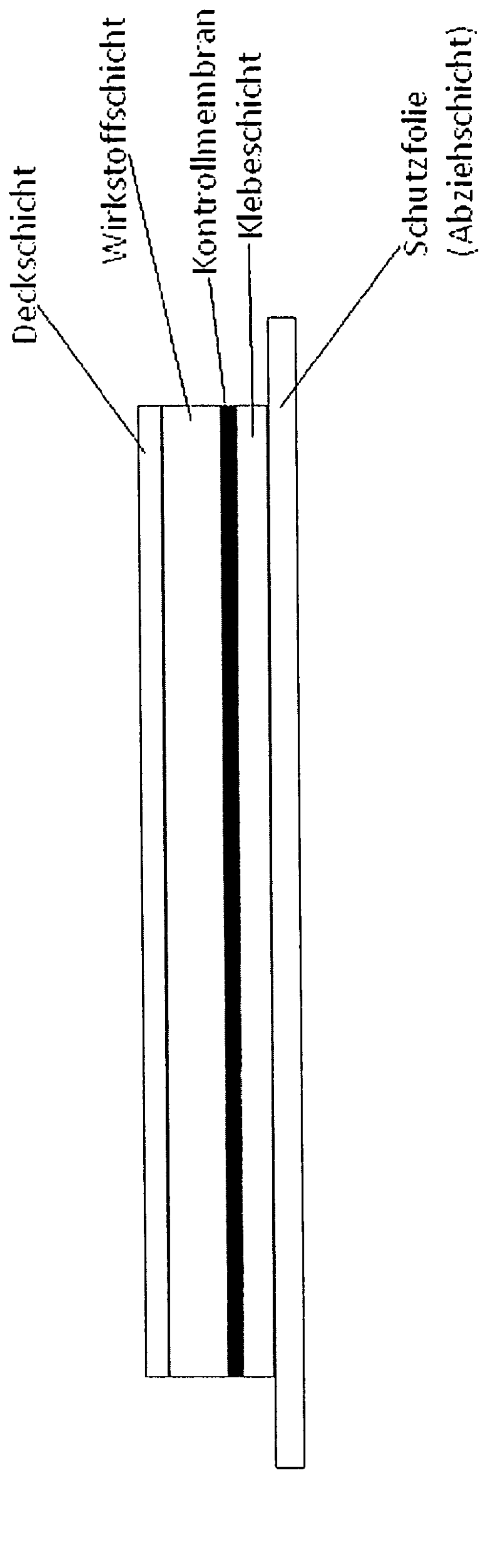
Deckschicht
Wirkstoffschicht
Kontrollmembran
Klebeschicht
Schutzfolie
(Abziehschicht)

Figur 2

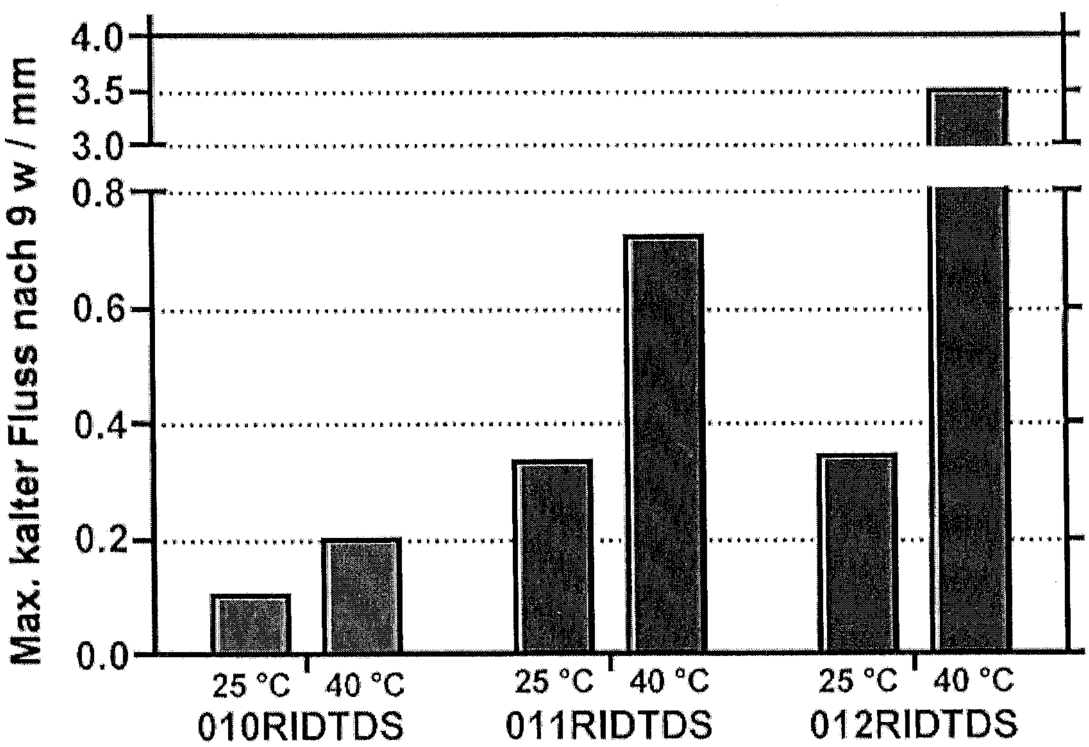

Max. kalter Fluss nach 9 w / mm
4.0
3.5
3.0
0.8
0.6
0.4
0.2
0.0
25 °C
40 °C
010RIDTDS
25 °C
40 °C
011RIDTDS
25 °C
40 °C
012RIDTDS

Figur 3

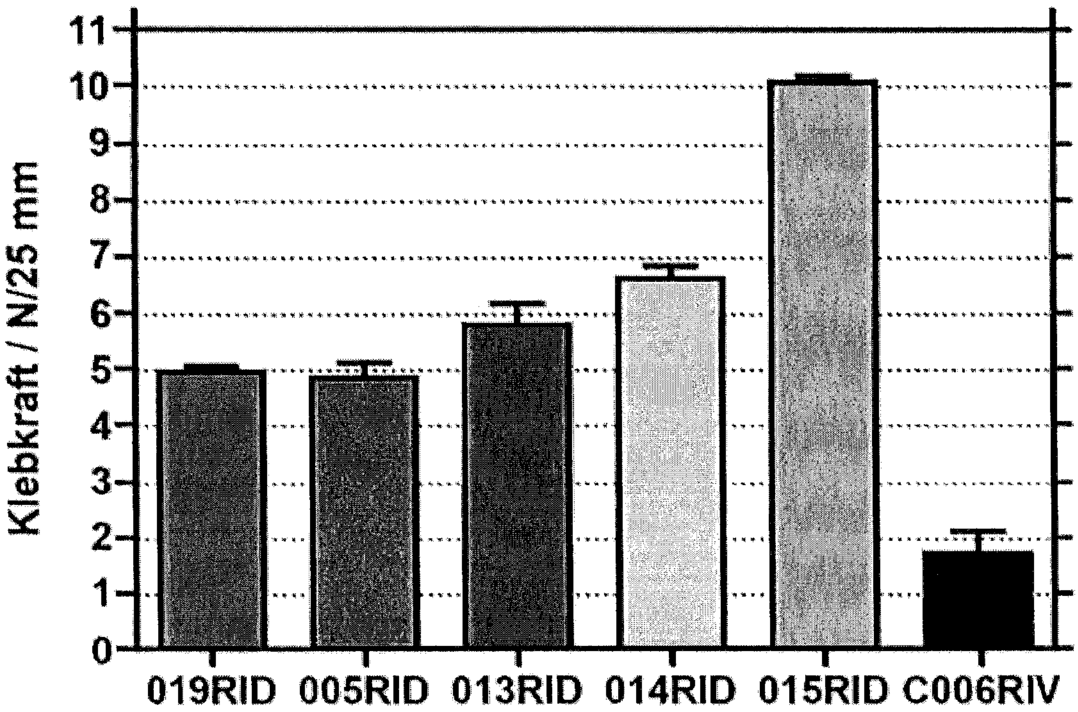
Klebkraft / N/25 mm
0
1
2
3
4
5
6
7
8
9
10
11
019RID
005RID
013RID
014RID
015RID
C006RIV

Figur 4

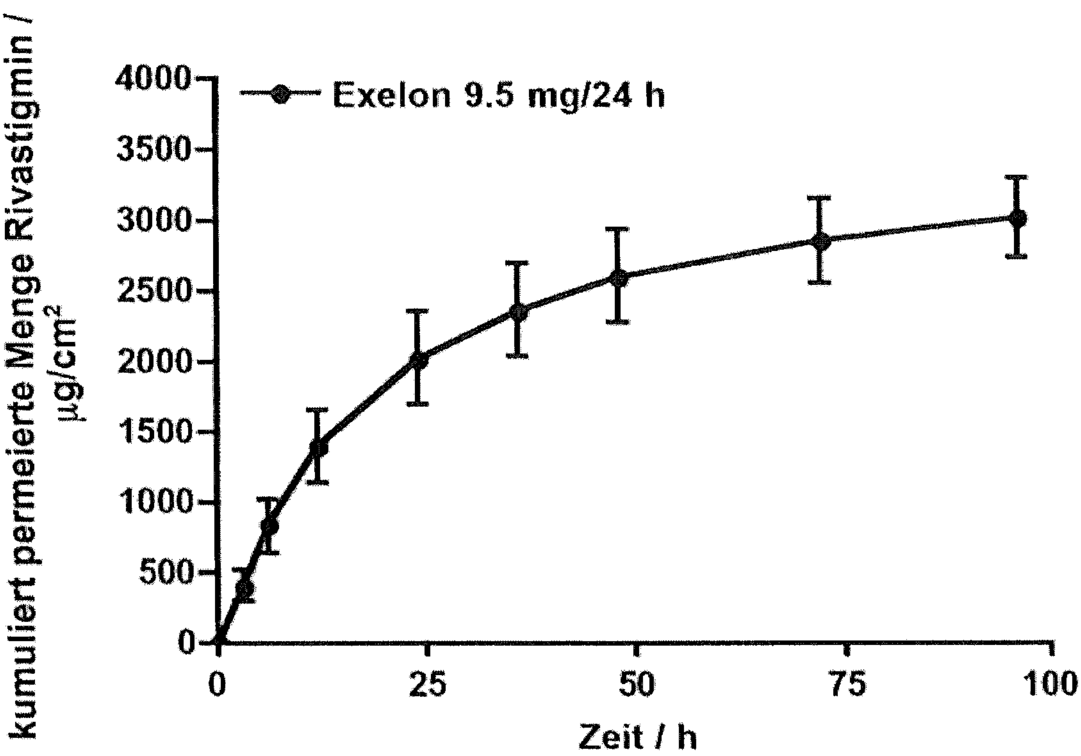
kumuliert permeierte Menge Rivastigmin / µg/cm²
4000
3500
3000
2500
2000
1500
1000
500
0
Exelon 9.5 mg/24 h
0
25
50
75
100
Zeit / h

Figur 5

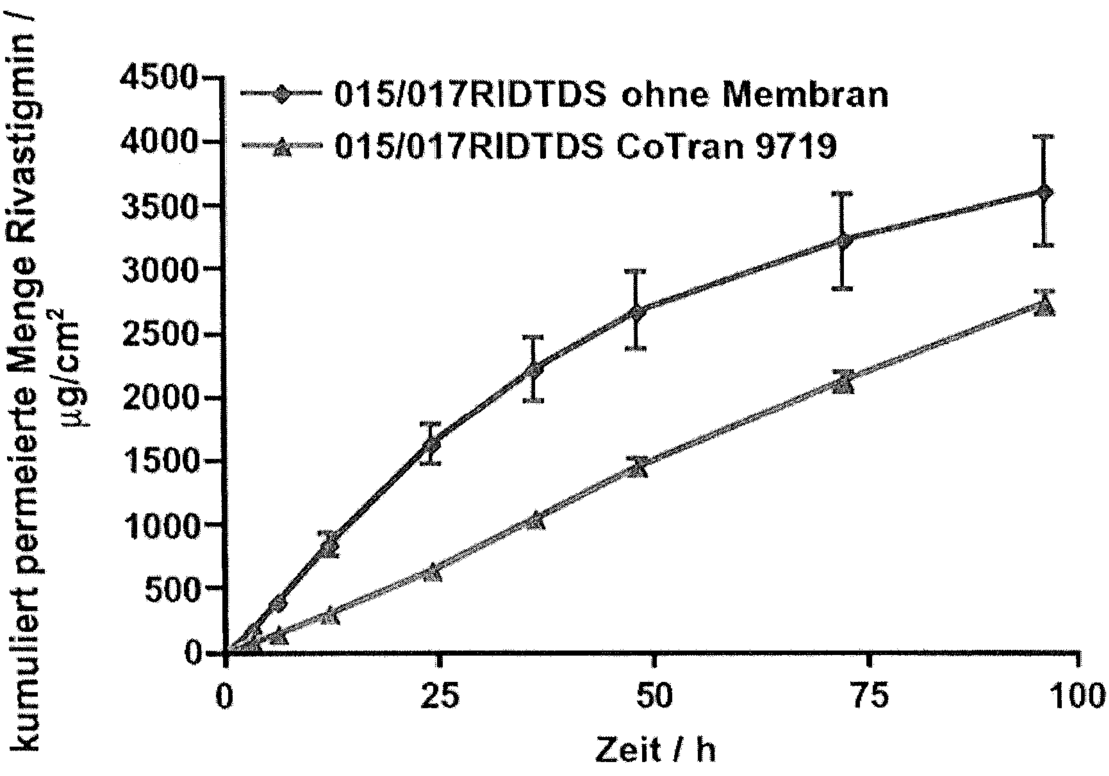
kumuliert permeierte Menge Rivastigmin / µg/cm²
4500
4000
3500
3000
2500
2000
1500
1000
500
0
015/017RIDTDS ohne Membran
015/017RIDTDS CoTran 9719
0
25
50
75
100
Zeit / h

Figur 6

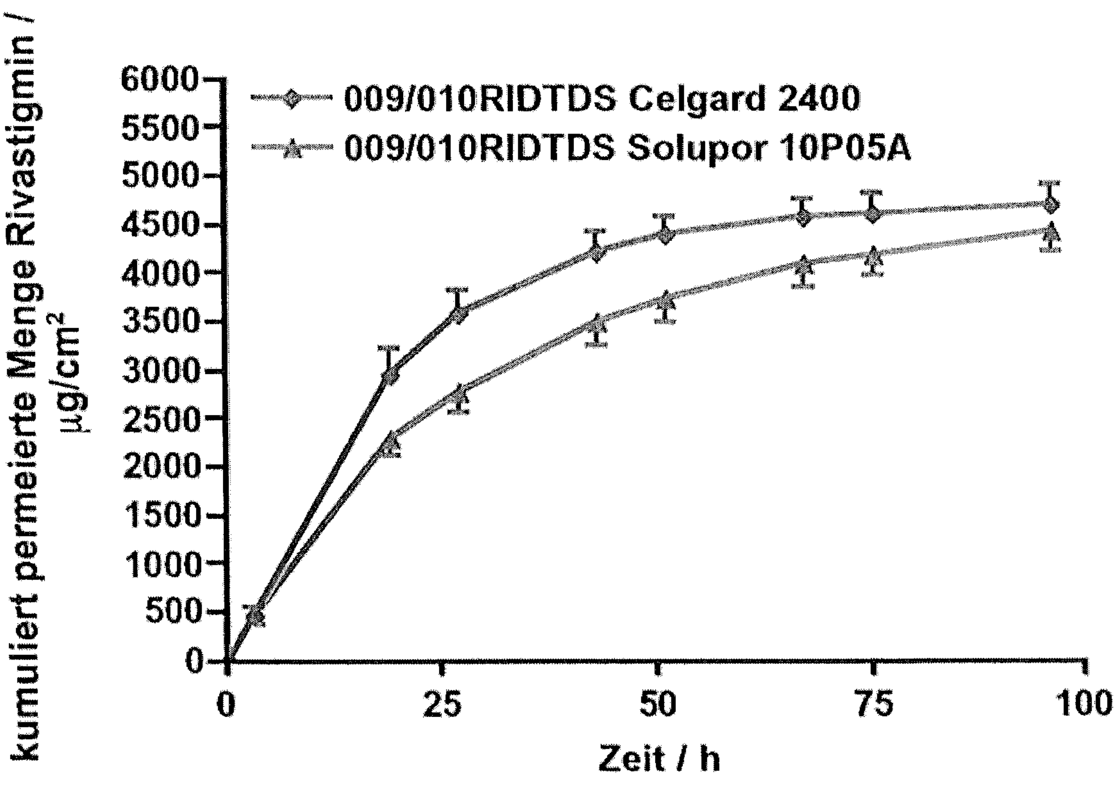
kumuliert permeierte Menge Rivastigmin / µg/cm2
6000
5500
5000
4500
4000
3500
3000
2500
2000
1500
1000
500
0
0
25
50
75
100
Zeit / h
009/010RIDTDS Celgard 2400
009/010RIDTDS Solupor 10P05A

Figur 7

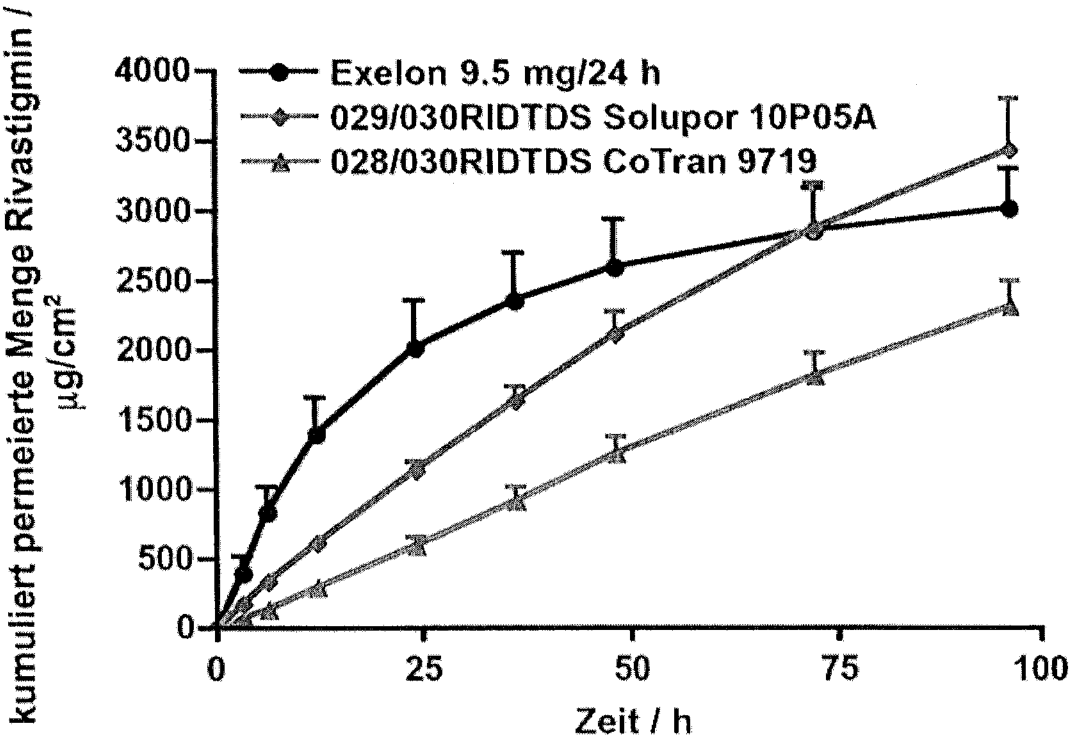
kumuliert permeierte Menge Rivastigmin / µg/cm²
4000
3500
3000
2500
2000
1500
1000
500
0
0
25
50
75
100
Zeit / h
Exelon 9.5 mg/24 h
029/030RIDTDS Solupor 10P05A
028/030RIDTDS CoTran 9719

Figur 8

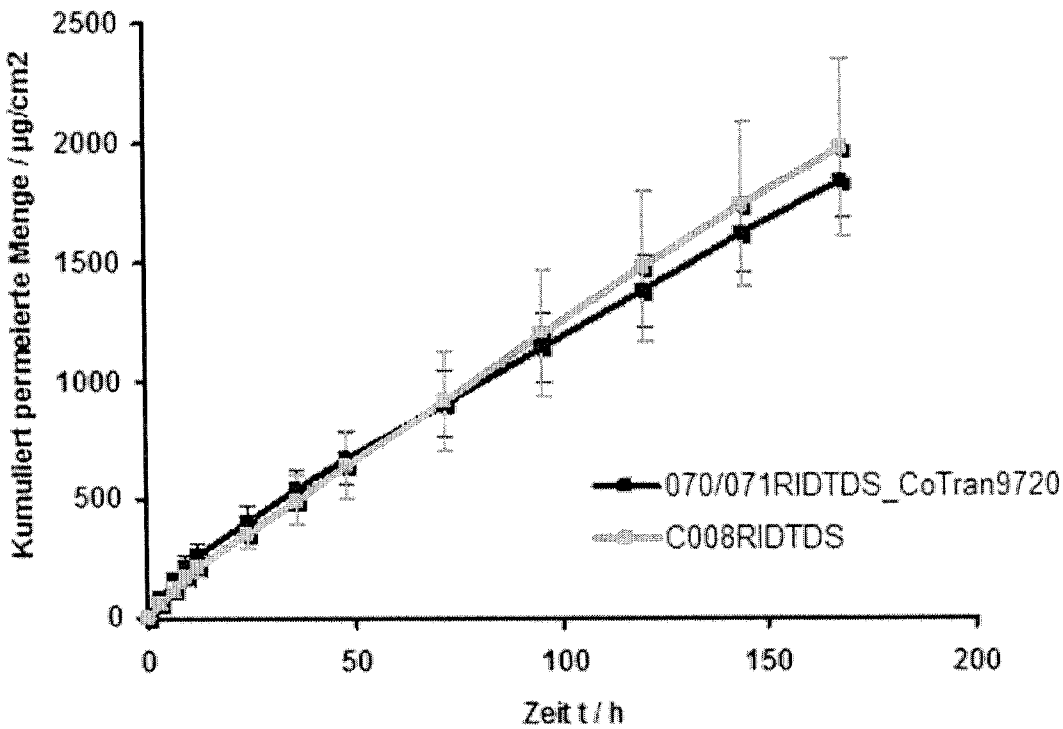
2500
2000
1500
1000
500
0
Kumuliert permeierte Menge / µg/cm2
0
50
100
150
200
Zeit t / h
070/071RIDTDS_CoTran9720
C008RIDTDS

Figur 9

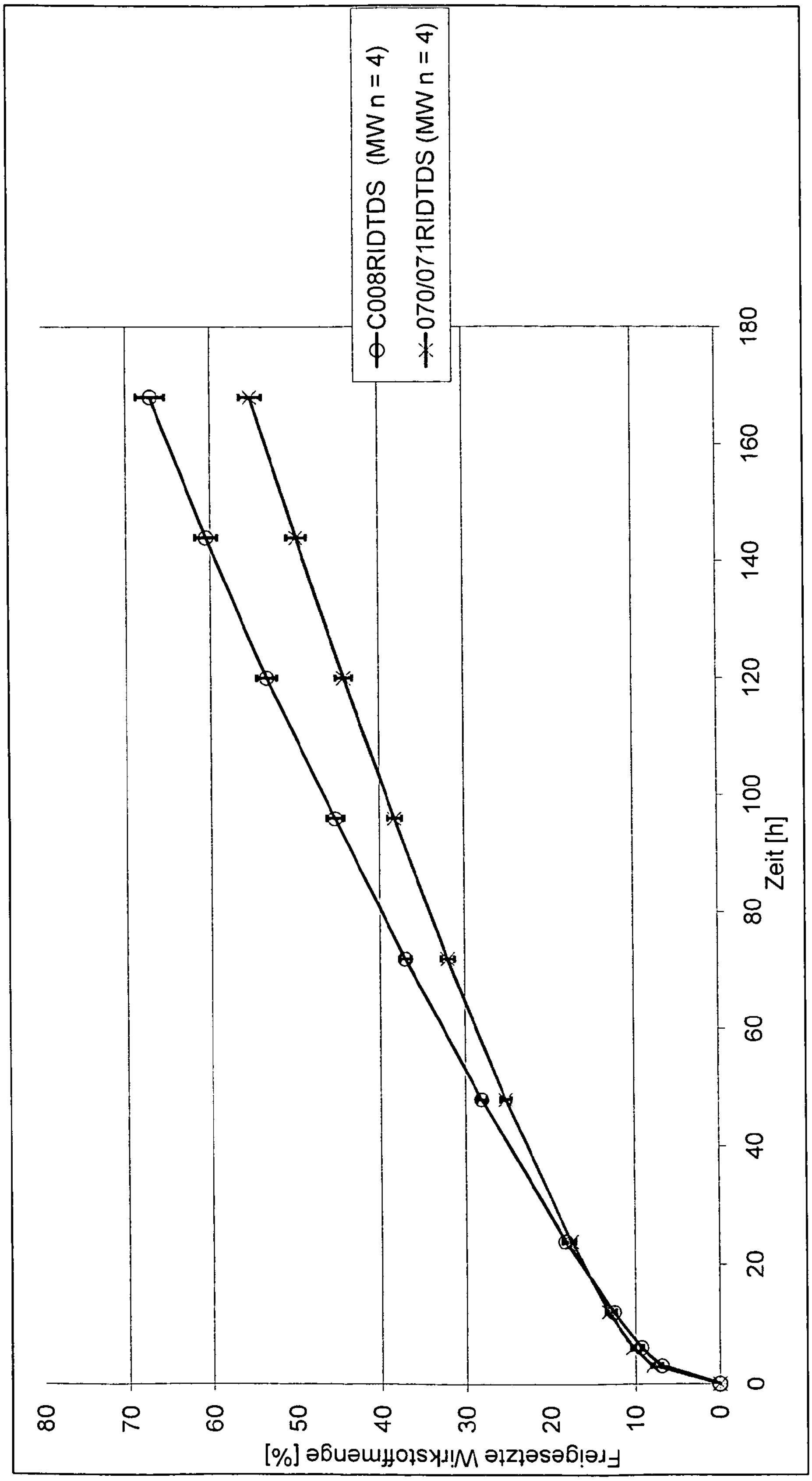
C008RIDTDS (MW n = 4)
070/071RIDTDS (MW n = 4)
Freigesetzte Wirkstoffmenge [%]
Zeit [h]
0
10
20
30
40
50
60
70
80
0
20
40
60
80
100
120
140
160
180

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 2203040 A
- WO 0203969 A
- WO 2008021113 A
- EP 2016939 A
- DE 19918106
- WO 2007064407 A1
- US 20080044461 A1
- US 20070259028 A1
- US 20040086552 A1
- US 6689379 B1
- EP 1047409 A
- WO 03017988 A1
- WO 03017988 A


### In der Beschreibung aufgeführte Nicht-Patentliteratur


- OECD GUIDELINE FOR THE TESTING OF CHEMICALS. Skin Absorption: in vitro method. *Test Guideline,* 13. April 2004, vol. 428, 1-8